# EUROPEAN PATENT SPECIFICATION

(11) **EP 2 013 201 B1**
(45) Date of publication and mention of the grant of the patent: **25.07.2012**
(21) Application number: 07733606.3
(22) Date of filing: 05.04.2007
(51) Int. Cl.: C07D 413/06, C07D 413/12, C07D 413/14, A61K 31/422

(54) **HETEROCYCLIC GPCR AGONISTS**
HETEROCYCLISCHE GPCR-AGONISTEN
AGONISTES GPCR HÉTÉROCYCLIQUES

(30) Priority: 06.04.2006 GB 0606913; 04.01.2007 GB 0700112
(43) Date of publication of application: 14.01.2009
(73) Proprietor: PROSIDION LTD, Oxford, Oxfordshire OX4 6LT (GB)
(72) Inventor: BERTRAM, Lisa Sarah, Oxford Oxfordshire OX4 6LT (GB); FYFE, Matthew Colin Thor, Oxford Oxfordshire OX4 6LT (GB); PROCTER, Martin James, Oxford Oxfordshire OX4 6LT (GB); WILLIAMS, Geoffrey, Martyn, Oxford Oxfordshire OX4 6LT (GB)
(74) Representative: Rands, Peter David
(86) International application number: PCT/GB2007/050183
(87) International publication number: WO 2007/116229

(56) References cited:
- WO-A-2005/061489
- WO-A-2007/003960
- US-A1- 2004 204 461
- OVERTON H. A. ET AL: "Deorphanization of a G protein-coupled receptor for oleoylethanolamide and its use in the discovery of small-molecule hypophagic agents", CELL METABOLISM, 2006, pages 167-175,

## Description

### BACKGROUND OF THE INVENTION

The present invention is directed to G-protein coupled receptor (GPCR) agonists. In particular, the present invention is directed to agonists of GPR119 that are useful for the treatment of obesity, e.g. as regulators of satiety, metabolic syndrome and for the treatment of diabetes.

Obesity is characterized by an excessive adipose tissue mass relative to body size. Clinically, body fat mass is estimated by the body mass index (BMI; weight(kg)/height(m)²), or waist circumference. Individuals are considered obese when the BM1 is greater than 30 and there are establislied medical consequences of being overweight. It has been an accepted medical view for some time that an increased body weight, especially as a result of abdominal body fat, is associated with an increased risk for diabetes, hypertension, heart disease, and numerous other health complications, such as arthritis, stroke, gallbladder disease, muscular and respiratory problems, back pain and even certain cancers.

Pharmacological approaches to the treatment of obesity have been mainly concerned with reducing fat mass by altering the balance between energy intake and expenditure. Many studies have clearly established the link between adiposity and the brain circuitry involved in the regulation of energy homeostasis. Direct and indirect evidence suggest that serotonergic, dopaminergic, adrenergic, cholinergic, endocannabinoid, opioid, and histaminergic pathways in addition to many neuropeptide pathways (e.g. neuropeptide Y and melanocortins) are implicated in the central control of energy intake and expenditure. Hypothalamic centres are also able to sense peripheral hormones involved in the maintenance of body weight and degree of adiposity, such as insulin and leptin, and fat tissue derived peptides.

Drugs aimed at the pathophysiology associated with insulin dependent Type I diabetes and non-insulin dependent Type 11 diabetes have many potential side effects and do not adequately address the dyslipidaemia and hyperglycaemia in a high proportion of patients. Treatment is often focused at individual patient needs using diet, exercise, hypoglycaemic agents and insulin, but there is a continuing need for novel antidiabetic agents, particularly ones that may be better tolerated with fewer adverse effects.

Similarly, metabolic syndrome (syndrome X) places people at high risk of coronary artery disease, and is characterized by a cluster of risk factors including central obesity (excessive fat tissue in the abdominal region), glucose intolerance, high triglycerides and low HDL cholesterol, and high blood pressure. Myocardial ischemia and microvascular disease is an established morbidity associated with untreated or poorly controlled metabolic syndrome.

There is a continuing need for novel antiobesity and antidiabetic agents, particularly ones that are well tolerated with few adverse effects.

GPR119 (previously referred to as GPR116) is a GPCR identified as SNORF25 in WO00/50562 which discloses both the human and rat receptors, US 6,468,756 also discloses the mouse receptor (accession numbers: AAN95194 (human), AAN95195 (rat) and ANN95196 (mouse)).

In humans, GPR119 is expressed in the pancreas, small intestine, colon and adipose tissue. The expression profile of the human GPR119 receptor indicates its potential utility as a target for the treatment of obesity and diabetes.

International patent applications WO2005/061489, WO2006/070208 WO2006/067532 disclose heterocyclic derivatives as GPR119 receptor agonists. International patent application WO2006/067531 discloses GPR119 receptor agonists. International patent applications WO2007/003960, WO2007/003961, WO2007/003962 and WO2007/003964, published after the priority date of the present application, also disclose GPR119 receptor agonists.

The present invention relates to agonists of GPR119 which are useful for the treatment of diabetes and as peripheral regulator of satiety, e.g. for the treatment of obesity and metabolic syndrome.

### SUMMARY OF THE INVENTION

Compounds of formula (I): 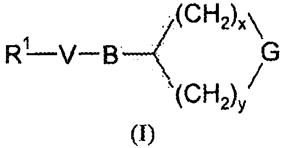 wherein V is a 5-membered heteroaryl ring of the formula: 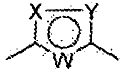 wherein W is N, and one of X and Y is N and the other is O;
B is (CH₂)ₚ-CH(C₁₋₃ alkyl)-(CH₂)_{q}, where one of the CH₂ groups may be replaced by O, NR⁵, S(O)ₘ, C(O), C(O)NR⁵ or CH(NR⁵R⁵⁵);
m is independently 0, 1 or 2;
p + q equals 0, 1 or 2;
x is 2;
y is 2;
G is NR²;
R¹ is phenyl or a 6-membered heteroaryl group containing up to two N atoms, any of which may be optionally substituted by one or more substituents selected from halo, C₁₋₄ alkyl, C₁₋₄ fluoroalkyl, C₁₋₄ hydroxyalkyl, C₃₋₇ cycloalkyl, OR⁶, CN, S(O)ₘR⁶, C(O)NR⁶R⁶⁶, NR⁶R⁶⁶, NR¹⁰C(O)R⁶, NR¹⁰SO₂R⁶, SO₂NR⁶R⁶⁶, COR¹⁰, C(O)OR¹⁰, a 4- to 7-membered heterocyclyl group or a 5- or 6-membered heteroaryl group;
R² is C(O)OR³, C(O)NR³R¹³, S(O)₂R³ or C(O)R³; or monocyclic 5- or 6- membered heteroaryl ring containing upto 4 heteroatoms selected from N, O and S, which may optionally be substituted by one or two groups selected from C₁₋₄alkyl, C₁₋₄alkoxy and halogen;
R³ is C₁₋₈ alkyl, C₂₋₈ alkenyl or C₂₋₈ alkynyl, any of which may be optionally substituted by one or more halo atoms, NR⁴R⁴⁴, OR⁴, C(O)OR⁴, OC(O)R⁴ or CN groups, and may contain a CH₂ group that is replaced by O or S; or C₃₋₇ cycloalkyl, aryl, 4 to 7 memembered heterocyclyl ring containing up to 3 heteroatoms selected from N, O and S, 6 membered heteroaryl ring containing up to 4 heteroatoms selected from N, O or S, C₁₋₄ alkylene, C₃₋₇ cycloalkyl, C₁₋₄ alkylenearyl, C₁₋₄ alkyleneheterocyclyl or C₁₋₄ alkyleneheteroaryl, any of which may be substituted with one or more substituents selected from halo, C₁₋₄ alkyl, C₁₋₄ fluoroalkyl, OR⁴, CN, NR⁴R⁴⁴, SO₂Me, NO₂ or C(O)OR⁴;
R⁴ and R⁴⁴ are independently hydrogen or C₁₋₄alkyl; or, taken together, R⁴ and R⁴⁴ may form a 5- or 6-membered heterocyclic ring;
R⁵ and R⁵⁵ independently represent hydrogen or C₁₋₄ alkyl;
R⁶ and R⁶⁶ are independently hydrogen or C₁₋₄ alkyl, which may optionally be substituted by halo, hydroxy, C₁₋₄ alkyloxy-, C₁₋₄ alkylthio-, C₃₋₇ heterocyclyl or N(R¹⁰)₂; or C₃₋₇ cycloalkyl, aryl, heterocyclyl or heteroaryl, wherein the cyclic groups may be substituted with one or more substituents selected from halo, C₁₋₄ alkyl, C₁₋₄ fluoroalkyl, OR⁹, CN, SO₂CH₃, N(R¹⁰)₂ and NO₂; or, taken together, R⁶ and R⁶⁶ may form a 5- or 6-membered heterocyclic ring optionally substituted by hydroxy, C₁₋₄ alkyl or C₁₋₄ hydroxyalkyl; or R⁶⁶ is C₁₋₄ alkyloxy-;
R⁹ is hydrogen, C₁₋₂ alkyl or C₁₋₂ fluoroalkyl;
R¹⁰ are independently hydrogen or C₁₋₄ alkyl; or a group N(R¹⁰)₂ may form a 4- to 7-membered heterocyclic ring optionally containing a further heteroatom selected from O and NR¹⁰; and
R¹³ is hydrogen or C₁₋₄ alkyl.

The molecular weight of the compounds of formula (I) is preferably less than 800, more preferably less than 600, especially less than 500.

In the compounds of formula (I) V is a 5-membered heteroaryl ring of the formula: 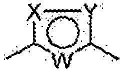 wherein W is N, and one of X and Y is N and the other is O.

Particular heterocyclic rings which V may represent include oxadiazole, oxazole, isoxazole, thiadiazole, thiazole and pyrazole.

In B, p + q is preferably 1 or 2, especially 1,
p is preferably 0 and q is preferably 1.

When one of the CH₂ groups in B is replaced, it is preferably replaced by O or NR⁵, more preferably by O. In one embodiment of the invention a CH₂ group in B is replaced. In a second embodiment of the invention a CH₂ group in B is not replaced.

In one embodiment of the invention, the -CH(C₁₋₃ alkyl)- group in B is -CH(CH₃)-. In a further embodiment B is -CH(CH₃)-O-. In compounds where B is -CH(CH₃)-O- the absolute configuration at the chiral carbon atom is preferably (R).

R¹ is phenyl or a 6-membered heteroaryl group containing up to two N atoms either of which rings may optionally be substituted, especially optionally substituted phenyl. Examples of R¹ heteroaryl groups include oxazolyl, isoxazolyl, thienyl, pyrazolyl, imidazolyl, furanyl, pyridazinyl or pyridyl. Preferred substituent groups for R¹ are halo, C₁₋₄ alkyl, C₁₋₄ fluoroalkyl, C₂₋₄ alkenyl, C₂₋₄ alkynyl, CN, S(O)ₘR⁶, C(O)NR⁶R⁶⁶, SO₂NR⁶R⁶⁶, COR¹⁰, C(O)OR¹⁰ or a 5- or 6-membered heteroaryl group; especially halo, e.g. fluoro or chloro, C₁₋₄ alkyl, C₁₋₄ fluoroalkyl, C₂₋₄ alkenyl, C₂₋₄ alkynyl, CN, S(O)ₘR⁶, C(O)NR⁶R⁶⁶ or SO₂NR⁶R⁶⁶ or a 5- or 6-membered heteroaryl group; in particular fluoro, chloro, methyl, S(O)ₘR⁶, e.g. where m is 1 or 2, C(O)NR⁶R⁶⁶ or SO₂NR⁶R⁶⁶.

G is NR².

x and y each represent 2.

R² is C(O)OR³, C(O)NR³R¹³, monocyclic 5- or 6- membered, heteroaryl group containing up to 4 heteroatoms selected from N, O and S, which may optionally be sustituted by one or two groups selected from C₁₋₄ alkyl, C₁₋₄ alkoxy and halogen; S(O)₂R³, or C(O)R³. More preferably, R² is C(O)OR³, C(O)NR³R¹³ or heteroaryl as defined above. R² is most preferably C(O)OR³. When R² is heteroaryl the heteroaryl ring is preferably pyrimidinyl, especially pyrimidin-2-yl, R² is more preferably oxadiazole.

Preferably R³ represents C₁₋₈ alkyl, C₂₋₈ alkenyl or C₂₋₈ alkynyl, optionally substituted by one or more halo atoms, NR⁴R⁴⁴, OR⁴, C(O)OR⁴, OC(O)R⁴ or CN groups, and may contain a CH₂ group that is replaced by O or S; or a C₃₋₇ Cycloalkyl, aryl or C₁₋₄ alkyl, or C₃₋₇ cycloalkyl, any of which may be substituted with one or more substituents selected from halo, C₁₋₄ alkyl, C₁₋₄ fluoroalkyl, OR⁴, CN, NR⁴R⁴⁴, NO₂ or C(O)OC₁₋₄ alkyl. More preferably R³ represents C₁₋₈ alkyl, C₂₋₈ alkenyl or C₂₋₈ alkynyl optionally substituted by one or more halo atoms or CN, and may contain a CH₂ group that may be replaced by O or S; or a C₃₋₇ cycloalkyl or aryl, either of which may be substituted with one or more substituents selected from halo, C₁₋₄ alkyl, C₁₋₄ fluoroalkyl, OR⁴, CN, NR⁴R⁴⁴, NO₂ or C(O)OC₁₋₄ alkyl. Most preferred R³ groups are C₃₋₅ alkyl optionally substituted by one or more halo atoms or CN, and may contain a CH₂ group that is replaced by O or S, or C₃₋₅cycloalkyl optionally substituted by C₁₋₄ alkyl. In one embodiment of the invention the group represented by R³ is unsubstituted.

R⁵ and R⁶⁶ are preferably optionally substituted C₁₋₄ alkyl or optionally substituted C₃₋₇ cycloalkyl.

When the group R⁶ is attached to a sulfinyl or sulfonyl group, R⁶ is preferably optionally substituted C₁₋₄ alkyl or optionally substituted C₃₋₇ cycloalkyl, more preferably optionally substituted C₁₋₄ alkyl, e.g. methyl or ethyl. When the group R⁶ is attached to C(O)N, R⁶ is preferably hydrogen, optionally substituted C₁₋₄ alkyl or optionally substituted C₃₋₇ cycloalkyl, more preferably optionally substituted C₁₋₄ alkyl, e.g. methyl or ethyl.

R⁹ is preferably C₁₋₂ alkyl or C₁₋₂ fluoroalkyl.

While the preferred groups for each variable have generally been listed above separately for each variable, preferred compounds of this invention include those in which several or each variable in formula (I) is selected from the preferred, more preferred or particularly listed groups for each variable. Therefore, this invention is intended to include all combinations of preferred, more preferred and particularly listed groups.

Specific compounds of the invention which may be mentioned are those included in the Examples and pharmaceutically acceptable salts thereof.

As used herein, unless stated otherwise, "alkyl" as well as other groups having the prefix "alk" such as, for example, alkenyl, alkynyl, and the like, means carbon chains which may be linear or branched or combinations thereof. Examples of alkyl groups include methyl, ethyl, propyl, isopropyl, butyl, sec- and tert-butyl, pentyl, hexyl, heptyl and the like. "Alkenyl", "alkynyl" and other like terms include carbon chains having at least one unsaturated carbon-carbon bond.

The term "fluoroalkyl" includes alkyl groups substituted by one or more fluorine atoms, e.g. CH₂F, CHF₂ and CF₃.

The term "cycloalkyl" means carbocycles containing no heteroatoms, and includes monocyclic and bicyclic saturated and partially saturated carbocycles. Examples of cycloalkyl include cyclopropyl, cyclobutyl, cyclopentyl, cyclohexyl and cycloheptyl. Examples of partially saturated cycloalkyl groups include cyclohexene and indane. Cycloalkyl groups will typically contain 3 to 10 ring carbon atoms in total, e.g. 3 to 6, or 8 to 10.

The term "halo" includes fluorine, chlorine, bromine, and iodine atoms.

The term "aryl" includes phenyl and naphthyl, in particular phenyl.

Unless otherwise indicated the term "heterocyclyl" and "heterocyclic ring" includes 4-to 10-membered monocyclic and bicyclic saturated rings, e.g. 4- to 7-membered monocyclic saturated rings, containing up to three heteroatoms selected from N, O and S. Examples of heterocyclic rings include oxetane, tetrahydrofuran, tetrahydropyran, oxepane, oxocane, thietane, tetrahydrothiophene, tetrahydrothiopyran, thiepane, thiocane, azetidine, pyrrolidine, piperidine, azepane, azocane, [1,3]dioxane, oxazolidine, piperazine, and the like. Other examples of heterocyclic rings include the oxidised forms of the sulfur-containing rings. Thus, tetrahydrothiophene 1-oxide, tetrahydrothiophene 1,1-dioxide, tetrahydrothiopyran 1-oxide, and tetrahydrothiopyran 1,1-dioxide are also considered to be heterocyclic rings.

Examples of heterocyclic rings that R² may represent include azetidine, pyrrolidine, piperidine and piperazine. R² heterocyclyl groups may also contain additional heteroatoms, e.g. morpholine.

Unless otherwise stated, the term "Heteroaryl" includes mono- and bicyclic 5- to 10-membered, e.g. monocyclic 5- or 6-membered, heteroaryl rings containing up to 4 heteroatoms selected from N, O and S. Examples of such heteroaryl rings are furyl, thienyl, pyrrolyl, pyrazolyl, imidazolyl, oxazolyl, isoxazolyl, thiazolyl, isothiazolyl, triazolyl, oxadiazolyl, thiadiazolyl, tetrazolyl, pyridinyl, pyridazinyl, pyrimidinyl, pyrazinyl and triazinyl. Bicyclic heteroaryl groups include bicyclic heteroaromatic groups where a 5- or 6-membered heteroaryl ring is fused to a phenyl or another heteroaromatic group. Examples of such bicyclic heteroaromatic rings are benzofuran, benzothiophene, indole, benzoxazole, benzothiazole, indazole, benzimidazole, benzotriazole, quinoline, isoquinoline, quinazoline, quinoxaline and purine.

Compounds described herein may contain one or more asymmetric centers and may thus give rise to diastereomers and optical isomers. The present invention includes all such possible diastereomers as well as their racemic mixtures, their substantially pure resolved enantiomers, all possible geometric isomers, and pharmaceutically acceptable salts thereof. The above formula (I) is shown without a definitive stereochemistry at certain positions. The present invention includes all stereoisomers of formula (I) and pharmaceutically acceptable salts thereof. Further, mixtures of stereoisomers as well as isolated specific stereoisomers are also included. During the course of the synthetic procedures used to prepare such compounds, or in using racemization or epimerization procedures known to those skilled in the art, the products of such procedures can be a mixture of stereoisomers.

When a tautomer of the compound of formula (I) exists, the present invention includes any possible tautomers and pharmaceutically acceptable salts thereof, and mixtures thereof, except where specifically drawn or stated otherwise.

When the compound of formula (I) and pharmaceutically acceptable salts thereof exist in the form of solvates or polymorphic forms, the present invention includes any possible solvates and polymorphic forms. A type of a solvent that forms the solvate is not particularly limited so long as the solvent is pharmacologically acceptable. For example, water, ethanol, propanol, acetone or the like can be used.

The term "pharmaceutically acceptable salts" refers to salts prepared from pharmaceutically acceptable non-toxic bases or acids. When the compound of the present invention is acidic, its corresponding salt can be conveniently prepared from pharmaceutically acceptable non-toxic bases, including inorganic bases and organic bases. Salts derived from such inorganic bases include aluminum, ammonium, calcium, copper (ic and ous), ferric, ferrous, lithium, magnesium, potassium, sodium, zinc and the like salts. Particularly preferred are the ammonium, calcium, magnesium, potassium and sodium salts. Salts derived from pharmaceutically acceptable organic non-toxic bases include salts of primary, secondary, and tertiary amines, as well as cyclic amines and substituted amines such as naturally occurring and synthesized substituted amines. Other pharmaceutically acceptable organic non-toxic bases from which salts can be formed include arginine, betaine, caffeine, choline; *N',N'-*dibenzylethylenediamine, diethylamine, 2-diethylaminoethanol, 2-dimethylaminoethanol, ethanolamine, ethylenediamine, *N*-ethylmorpholine, *N*-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, its corresponding salt can be conveniently prepared from pharmaceutically acceptable non-toxic acids, including inorganic and organic acids. Such acids include, for example, acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid and the like

Since the compounds of formula (I) are intended for pharmaceutical use they are preferably provided in substantially pure form, for example at least 60% pure, more suitably at least 75% pure, especially at least 98% pure (% are on a weight for weight basis).

The compounds of formula (I) can be prepared as described below, in which, for illustrative purposes, -V- is shown as a group of the formula: 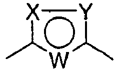 and R¹, A, B, x, y, G, W, X and Y are as defined above.

The compounds of formula (I), in which X = N, Y = O and W = N, may be prepared according to the method illustrated in Scheme 1. The nitriles of formula **2** are either commercially available or can be synthesised using known techniques. Compounds of formula **2** are treated with hydroxylamine in a suitable solvent, such as ethanol-water, at elevated temperature, to afford amidoximes of formula **3** (synthesis of amidoximes is further described by A. R. Martin et al, J. Med. Chem., 2001, 44, 1560). Compounds of formula **3** are subsequently condensed with acids of formula **4,** which are themselves either commercially available or can be readily synthesised using known techniques. The condensation firstly entails activation of compounds of formula **4** by, for example, formation of the mixed anhydride, in which the acid is treated with a chloroformate, such as isobutylchloroformate, in the presence of a suitable base, such as triethylamine, in a suitable solvent, such as THF or toluene, followed by addition of compounds of formula **3.** Alternatively, compounds of formula **4** may be activated by conversion to the acid halide, generated by treatment of the acid with, for example, oxalyl chloride in a suitable solvent, such as CH₂Cl₂-DMF. The intermediates arising from the condensation of amidoximes of formula 3 and acids of formula **4** are dissolved in an appropriate solvent, such as toluene or xylene, and heated under reflux, with concomitant removal of water by Dean-Stark apparatus or by molecular sieves, to form oxadiazoles of formula (I). Alternatively, amidoximes of formula **3** can firstly be treated with a suitable base, for example sodium hydride, in an appropriate solvent, such as THF, and subsequently esters of formula **5.** Heating of this mixture also generates oxadiazoles of formula (I) (this process is further illustrated by R. H. Mach et al, Bioorg. Med. Chem., 2001, 9, 3113).

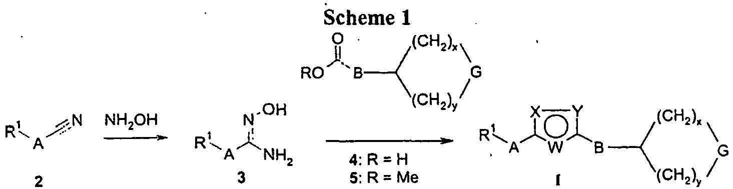

Compounds of formula (I) in which X = O, Y = N and W = N may be prepared according to the method outlined in Scheme 2. The nitriles of formula **6** are either commercially available or can be synthesised using known techniques. These are converted to the corresponding amidoximes of formula **7,** as described above, and subsequently condensed with acids of formula **8,** which are commercially available or can readily be synthesised by those skilled in the art. This condensation is performed in a fashion analogous to that described in Scheme 1, to afford the corresponding oxadiazoles of formula (I).

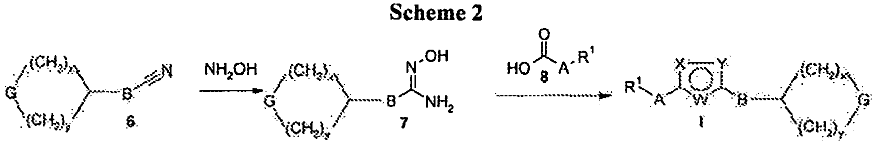

Compounds of formula (I) as disclosed herein in which X = N, Y = N and W = O can be synthesised as outlined in Scheme 3. The acyl chlorides of formula **9** are either commercially available or may be synthesised using known methods. The acid hydrazides of formula **10** can be readily obtained by, for example, treating an ethanolic solution of the corresponding ester with hydrazine (for further details see K. M. Kahn et al, Bioorg. Med. Chem., 2003, 11, 1381). Treating the acyl chlorides of formula **9** with the acid hydrazides of formula 10 in a suitable solvent, such as pyridine, affords compounds of formula **11** (further illustrated by V. N. Kerr et al, J. Am. Chem. Soc., 1960, 82, 186), which are then converted by POCl₃ at elevated temperature to compounds of formula (I) (this process is further described by S-A. Chen et al, J. Am. Chem. Soc., 2001, 123, 2296). Similarly, compounds of formula (I) where X = Y = W = N can be prepared via the condensation of the amidrazone analogue of **10** with the appropriate activated carboxylic acid derivative, such as **9.** The reactive groups in this reaction may be exchanged, i.e. an amidrazone of formula R¹-A-C(=NH)NHNH₂ can form a compound of formula (I) by condensation with an activated carboxylic acid derivative LG-C(=O)-B-cycle where LG is halogen or oxycarbonyl (P. H. Olesen et al, J. Med. Chem., 2003, 46, 3333-3341).

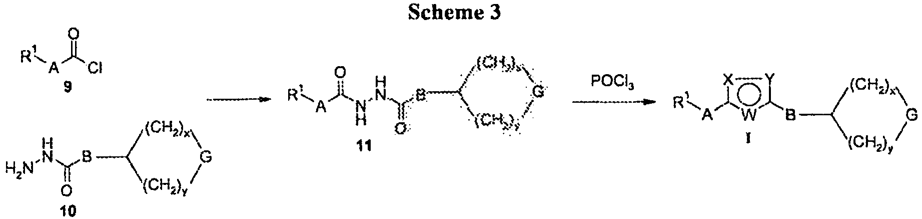

Compounds of formula (I) as disclosed herein where X = N, Y = N, and W = S can also be prepared from compounds of formula **11** by heating with Lawesson's reagent in a suitable solvent, such as toluene or acetonitrile (D. Alker et al, J. Med. Chem., 1989, 32, 2381-2388). Compounds of formula (I) where X = S, Y = N and W = N can be formed from compounds of formula **12** (Scheme 4) which are commercially available, or can be readily synthesised from the corresponding carbonyl compound and Lawesson's reagent under standard conditions. Treating a compound of formula **12** with a compound of formula **13** in a suitable solvent such as dichloromethane at about 20°C gives compounds of formula **14.** Compounds of formula **13** can be obtained by treating the corresponding dimethylamide with Meerwein's reagent (for details see M. Brown US 3,092,637). Compounds of formula **14** are then cyclised using hydroxylamine-*O*-sulfonic acid in the presence of a base, such as pyridine, in a suitable solvent such as methanol (for further details, see A. MacLeod et al, J. Med. Chem., 1990, 33, 2052).

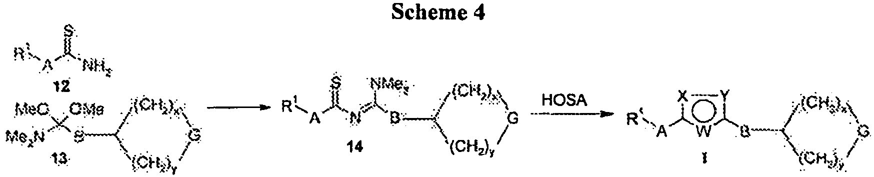

The regioisomeric derivatives of formula (I), where X = N, Y = S and W = N, can be formed in a similar manner by reversing the functionality of the reactants so the R¹ fragment contains the acetal moiety and the G containing cycle fragment contains the thiocarbonyl.

Compounds of formula (I) as disclosed herein where W = O, X = N and Y = CH can be formed from compounds of formula **15** (Scheme 5). Compounds of formula **15** are commercially available or synthesised using known techniques. Chlorides of formula **16** are commercially available, or can readily be formed by chlorinating the corresponding ketone using standard conditions, for example, bubbling chlorine gas through a methanol solution of the ketone (for further details see R. Gallucci & R. Going, J. Org. Chem., 1981, 46, 2532). Mixing a compound of formula **15** with a chloride of formula **16** in a suitable solvent, such as toluene, with heating, for instance at about 100°C gives compounds of formula (I) (for further information, see A. Hassner et al, Tetrahedron, 1989, 45, 6249). Compounds of formula (I) where W = O, X = CH and Y = N can be formed is a similar fashion by reversing the functionality of the reactants so the R¹ fragment contains the haloketone moiety and the G containing cycle fragment contains the C(O)NH₂.

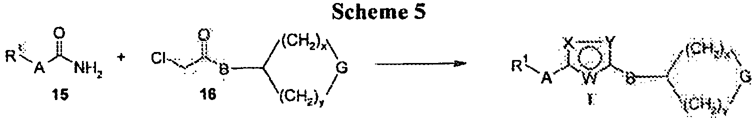

Alternatively, compounds of formula (I) which are disclosed herein where X = S, W = N and Y = CH can also be formed from compounds of formula **16.** Heating an compound of formula **15** with phosphorus pentasulfide, followed by the addition of a compound of **formula 16** followed by further heating gives compounds of formula (I) (for further details, see R. Kurkjy & E. Brown, J. Am. Chem. Soc., 1952, 74, 5778). The regioisomeric compounds where X = CH, W = N and Y = S can be formed is a similar fashion by reversing the functionality of the reactants, so the R¹ fragment contains the haloketone moiety and the G containing cycle fragment contains the C(O)NH₂.

Compounds of formula I which are disclosed herein where W = N, X = O and Y = CH can be formed from compounds of formula **15** and formula **17** (Scheme 6) under similar conditions to those outlined for Scheme 5. Compounds of formula I where W = S, X = N and Y = CH can also be formed from compounds of formula **15** and formula **17** using the conditions involving phosphorus pentasulfide described above.

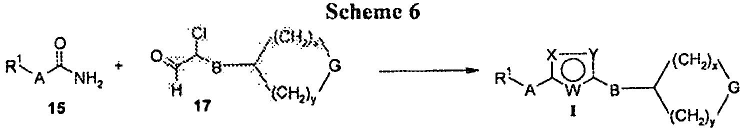

Compounds of formula (I) as disclosed herein where X = O, Y = N and W = CH, and where X = N, Y = O and W = CH and can be formed from compounds of formula **20** (Scheme 7). Acylation of compounds of formula **18** with a compound of formula **19,** where Q is alkoxide or chloride, can occur under standard conditions, for example, deprotonation of ketone **18** with a suitable base, such as lithium diisopropylamide or potassium ethoxide, in a suitable solvent, such as tetrahydrofuran, generally at low temperature. Treatment of compounds of formula **20** with hydroxylamine, in a suitable solvent, such as ethanol, at elevated temperature, for example 75°C, yields compounds of formula (I) as a mixture of both regioisomers of the isoxazole. Using standard separation techniques, such as chromatography on silica gel, the individual isomers can be isolated (for further details, see M. Rowley et al, J. Med. Chem., 1997, 40, 2374).

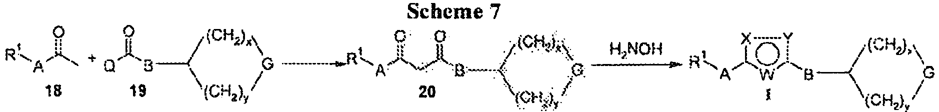

Compounds of formula (I) as disclosed herein where X = S, Y = N and W = CH can be formed by hydrogenation of a compound of formula (I) where X = O, Y = N and W = CH, with platinum oxide in a suitable solvent such as ethanol, followed by heating with phosphorus pentasulfide to give compounds of formula (I) where X = S, Y = N and W = CH (for further details, see G. Wiegand et al, J. Med. Chem., 1971, 14, 1015). For details of the synthesis of the regioisomer where X = N, Y = S and W = CH also see G. Wiegand *ibid.*

Compounds of formula (I) as disclosed herein where X = N, Y = N and W = CH can be formed from compounds of formula **20.** Treatment of compounds of formula **20** with hydrazine in a suitable solvent, such as methanol, would give rise to compounds of formula (I) where X = N, Y = N and W = CH (this process is further illustrated by R. Baker et al, J. Med. Chem., 1997, 40, 2374).

Compounds of formula (I) which are also disclosed herein in which X = CH, Y = N and W = N can be synthesised as described in Scheme 8. Bromides of formula **23** are either commercially available or may be synthesised from the corresponding ketone by, for example, treating an aqueous solution of the ketone with Br₂ and HBr (as described by J. Y. Becker et al, Tetrahedron Lett., 2001, 42, 1571). The amidines of formula **22** may be synthesised by known methods, for example by treatment of the corresponding alkyl imidates of formula **21** with ammonia in a suitable solvent, such as ethanol (as detailed by D. A. Pearson et al, J. Med. Chem., 1996, 39, 1372). The imidates of formula **21** may in turn be generated by, for example, treatment of the corresponding nitrile with HCl in a suitable solvent, such as methanol (for further details see J. P. Lokensgard et al, J. Org. Chem., 1985, 50, 5609). Reaction of amidines of formula **22** with bromides of formula **23** in a suitable solvent, such as DMF, affords compounds of formula (1) (illustrated by N. J. Liverton et al, J. Med. Chem., 1999, 42, 2180).

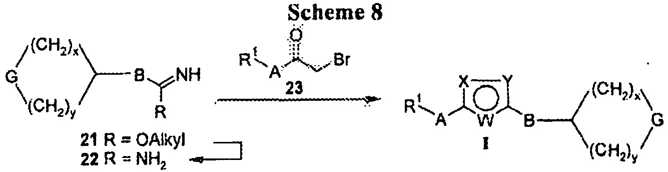

The regioisomeric compounds where X = N, Y = CH and W = N can be formed in a similar fashion by reversing the functionality of the reactants, so the R¹ fragment contains the amidine moiety and the R² fragment contains the bromide.

Compounds of formula (I) as disclosed herein in which X = CH, Y = CH and W = N can be synthesised as illustrated in Scheme 9. Diketones of formula **25** are readily accessible by, for example, the condensation of ketones of formula **24,** which are commercially available or are readily synthesised using known techniques, with bromides of formula **23** in a suitable solvent, such as benzene using an appropriate catalyst. Illustrative examples are described by O. G. Kulinkovich et al, Synthesis, 2000, 9, 1259. Using a Paal-Knorr reaction, diketones of formula **25** may be treated with, for example, ammonium carbonate in a suitable solvent, such as ethanol at elevated temperature (for further details see R. A. Jones et al, Tetrahedron, 1996, 52, 8707) to afford compounds of formula (I).

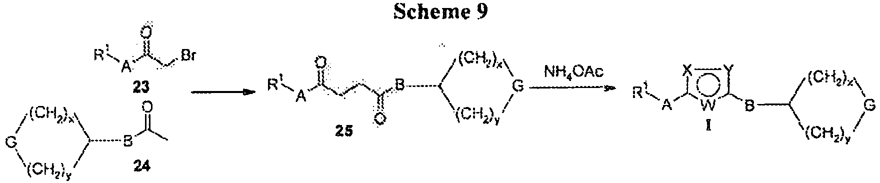

Compounds of formula (I) in which R² contains either a carbamate or a sulfonamide group may be synthesised as described in Scheme 10. Compounds of formula **26,** in which P represents a suitable protecting group, for example *tert*-butoxycarbonyl (Boc), may be synthesised as outlined in Schemes 1-9 above. The protecting group is firstly removed under suitable conditions to afford compounds of formula **27.** In the case of the Boc group this can be achieved by treatment of compounds of formula **26** with a suitable acid, such as trifluoroacetic acid, in an appropriate solvent, such as CH₂Cl₂. Treatment of compounds of formula **27** with chloroformates of formula **28,** which are generally commercially available or can be readily synthesised, in a suitable solvent, such as CH₂Cl₂, in the presence of a suitable base, such as triethylamine, affords compounds of formula (I). Similarly, compounds of formula **27** may be reacted with sulfonyl chlorides of formula **29,** which are generally commercially available or can readily be synthesised, in a suitable solvent, such as CH₂Cl₂, in the presence of a suitable base, such as triethylamine, to afford compounds of formula (I). Compounds of formula (I) in which R² contains a urea moiety may be prepared by reacting a compound of formula **27** with an isocyanate of formula O=C=N-R³. Furthermore, compounds of formula (1) in which R² a heteroaryl group may be prepared by reacting the amine **27** with the appropriate heteroaryl chloride or bromide under Pd(0) catalysis in the presence of a suitable ligand and base (Urgaonkar, S.; Hu, J.-H.; Verkade, J. G. J. Org. Chem., 2003, 68, 8416-8423).

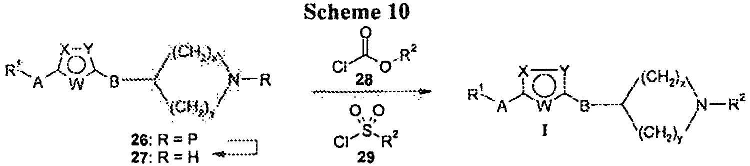

Compounds of formula (I) in which R² contains an amide group may be synthesised from compounds of formula **27** and a suitable acid (R³COOH), or activated derivative thereof, in an amide bond forming reaction.

Compounds of formula (I) where B contains a NR⁵ group where R⁵ is hydrogen can be further transformed into compounds of formula (I) where R⁵ is C₁₋₄ alkyl group using standard techniques known to those with skill in the art.

Compounds of the formula (I) where R¹ is pyridyl optionally substituted with CN can be prepared from the corresponding unsubstituted pyridine by the Reissert reaction (Fife, W. K. J. Org. Chem., 1983, 48, 1375-1377). Similar reactions can be used to prepare the compounds where R¹ is pyridyl optionally substituted with halogen (Walters, M. A.; Shay, J. J. Tetrahedron Lett., 1995, 36, 7575-7578). The compounds where R¹ is pyridyl optionally substituted with halogen can be transformed into the corresponding compounds where R¹ is pyridyl optionally substituted with C₁₋₄ alkyl by transition metal-catalysed cross-coupling reactions (Fürstner, A., et al, J. Am. Chem. Soc., 2002, 124, 13856-13863).

Compounds of formula (I) as disclosed herein where X = N, Y = N, U = N and W = CH can be synthesised as shown in Scheme 11 below. Illustrative examples are described by M. Meldal et al, J. Org. Chem., 2002, 67(9), 3057-3064. Azides of formula **30** are either commercially available or may be synthesised, for example, from the displacement of the corresponding halides with azide ion using known techniques; or synthesised from the corresponding amine derivative via reaction with sodium nitrite in acidic media. The alkynes of formula **31** may be commercial or synthesised by known methods, for example by reaction of acetylide ions with boranes (see J. Org. Chem., 1981, 46(11) 2311-2314) or aldehydes or ketones.

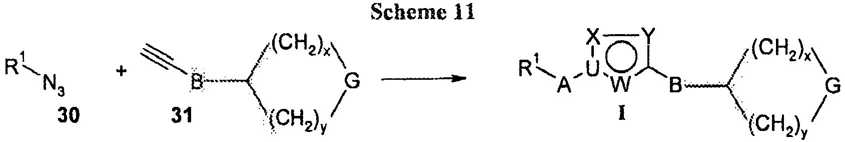

Compounds of formula (I) which are, for completeness, disclosed herein where X = N, Y = CH, U = N and W = CH can be synthesised as shown in Scheme 12 by reaction of 1,3-dicarbonyl compounds of formula **33** (or their equivalents, such as enol ethers) with hydrazines of formula **32**. The hydrazines of formula **32** may be commercial or synthesised by known methods, for example by reaction of the corresponding amine with sodium nitrite and reacting the resulting diazonium salt with a reducing agent such as sodium sulfite.

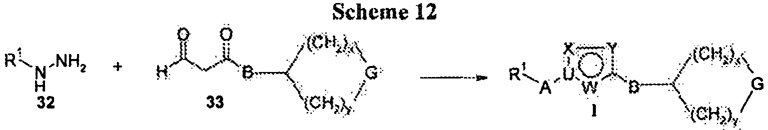

Other compounds of formula (I) may be prepared by methods analogous to those described above or by methods known *per se.*

Further details for the preparation of the compounds of formula (I) are found in the examples.

The compounds of formula (I) may be prepared singly or as compound libraries comprising at least 2, for example 5 to 1,000, compounds and more preferably 10 to 100 compounds of formula (I). Compound libraries may be prepared by a combinatorial "split and mix" approach or by multiple parallel synthesis using either solution or solid phase chemistry, using procedures known to those skilled in the art.

During the synthesis of the compounds of formula (I), labile functional groups in the intermediate compounds, e.g. hydroxy, carboxy and amino groups, may be protected. The protecting groups may be removed at any stage in the synthesis of the compounds of formula (I) or may be present on the final compound of formula (I). A comprehensive discussion of the ways in which various labile functional groups may be protected and methods for cleaving the resulting protected derivatives is given in, for example, Protective Groups in Organic Chemistry, T.W. Greene and P.G.M. Wuts, (1991) Wiley-Interscience, New York, 2nd edition.

Any novel intermediates as defined above are of use in the synthesis of compounds of formula (I) and are therefore also disclosed. For example, the invention provides compounds of formula (XXVII): 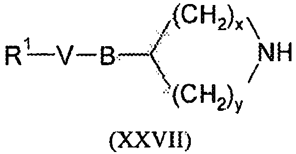 wherein V is a 5-membered heteroaryl ring of the formula: 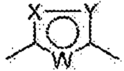 wherein W is N, and one of X and Y is N and the other is O;
B is (CH₂)ₚ-CH(C₁₋₃ alkyl)-(CH₂)_{q}, where one of the CH₂ groups may be replaced by O, NR⁵, S(O)ₘ, C(O), C(O)NR⁵, CH(NR⁵R⁵⁵), C(O)O, C(O)S, SC(O) or OC(O);
m is independently 0, 1 or 2;
p + q equals 0, 1 or 2;
x is 2;
y is 2;
R¹ is phenyl or a 6-membered heteroaryl group containing up to two N atoms, any of which may be optionally substituted by one or more substituents selected from halo, C₁₋₄ alkyl, C₁₋₄ fluoroalkyl, C₁₋₄ hydroxyalkyl, C₂₋₄ alkenyl, C₂₋₄ alkynyl, C₃₋₇ cycloalkyl, aryl, OR⁶, CN, NO₂, S(O)ₘR⁶, C(O)NR⁶R⁶⁶, NR⁶R⁶⁶, NR¹⁰C(O)R⁶, NR¹⁰SO₂R⁶, SO₂NR⁶R⁶⁶, COR¹⁰, C(O)OR¹⁰, a 4- to 7-membered heterocyclyl group or a 5- or 6-membered heteroaryl group, provided that R¹ is not 4-fluoroalkylpyrid-3-yl or 4-fluoroalkylpyrimidin-5-yl;
R⁵ and R⁵⁵ independently represent hydrogen or C₁₋₄ alkyl;
R⁶ and R⁶⁶ are independently hydrogen or C₁₋₄ alkyl, which may optionally be substituted by halo, hydroxy, C₁₋₄ alkyloxy-, C₁₋₄ alkylthio-, C₃₋₇ heterocyclyl or N(R¹⁰)₂; or C₃₋₇ cycloalkyl, aryl, heterocyclyl or heteroaryl, wherein the cyclic groups may be substituted with one or more substituents selected from hallo, C₁₋₄ alkyl, C₁₋₄ fluoroalkyl, OR⁹, CN, SO₂CH₃, N(R¹⁰)₂ and NO₂; or, taken together, R⁶ and R⁶⁶ may form a 5- or 6-membered heterocyclic ring optionally substituted by hydroxy, C₁₋₄ alkyl or C₁₋₄ hydroxyalkyl; or R⁶⁶ is C₁₋₄ alkyloxy-; and R¹⁰ are independently hydrogen or C₁₋₄ alkyl; or a group N(R¹⁰)₂ may form a 4- to 7-membered heterocyclic ring optionally containing a further heteroatom selected from O and NR¹⁰.

US 2003/0162812 discloses certain 4-fluoroalkylpyrid-3-yl and 4-fluoroalkylpyrimidin-5-yl derivatives as pesticides.

As indicated above the compounds of formula (I) are useful as GPR119 agonists, e.g. for the treatment and/or prophylaxis of obesity and diabetes. For such use the compounds of formula (1) will generally be administered in the form of a pharmaceutical composition.

The invention also provides a compound of formula (I), or a pharmaceutically acceptable salt thereof, for use as a pharmaceutical.

The invention also provides a pharmaceutical composition comprising a compound of formula (I), in combination with a pharmaceutically acceptable carrier.

Preferably the composition is comprised of a pharmaceutically acceptable carrier and a non-toxic therapeutically effective amount of a compound of formula (I), or a pharmaceutically acceptable salt thereof.

Moreover, we disclose a pharmaceutical composition for the treatment of disease by modulating GPR119, resulting in the prophylactic or therapeutic treatment of obesity, e.g. by regulating satiety, or for the treatment of diabetes, comprising a pharmaceutically acceptable carrier and a non-toxic therapeutically effective amount of compound of formula (I), or a pharmaceutically acceptable salt thereof.

The pharmaceutical compositions may optionally comprise other therapeutic ingredients or adjuvants. The compositions include compositions suitable for oral, rectal, topical, and parenteral (including subcutaneous, intramuscular, and intravenous) administration, although the most suitable route in any given case will depend on the particular host, and nature and severity of the conditions for which the active ingredient is being administered,

The pharmaceutical compositions may be conveniently presented in unit dosage form and prepared by any of the methods well known in the art of pharmacy.

In practice, the compounds of formula (I), or pharmaceutically acceptable salts thereof, can be combined as the active ingredient in intimate admixture with a pharmaceutical carrier according to conventional pharmaceutical compounding techniques. The carrier may take a wide variety of forms depending on the form of preparation desired for administration, e.g. oral or parenteral (including intravenous).

Thus, the pharmaceutical compositions can be presented as discrete units suitable for oral administration such as capsules, cachets or tablets each containing a predetermined amount of the active ingredient. Further, the compositions can be presented as a powder, as granules, as a solution, as a suspension in an aqueous liquid, as a non-aqueous liquid, as an oil-in-water emulsion, or as a water-in-oil liquid emulsion. In addition to the common dosage forms set out above, the compound of formula (I), or a pharmaceutically acceptable salt thereof, may also be administered by controlled release means and/or delivery devices. The compositions may be prepared by any of the methods of pharmacy. In general, such methods include a step of bringing into association the active ingredient with the carrier that constitutes one or more necessary ingredients. In general, the compositions are prepared by uniformly and intimately admixing the active ingredient with liquid carriers or finely divided solid carriers or both. The product can then be conveniently shaped into the desired presentation.

The compounds of formula (I), or pharmaceutically acceptable salts thereof, can also be included in pharmaceutical compositions in combination with one or more other therapeutically active compounds.

The pharmaceutical carrier employed can be, for example, a solid, liquid, or gas. Examples of solid carriers include lactose, terra alba, sucrose, talc, gelatin, agar, pectin, acacia, magnesium stearate, and stearic acid. Examples of liquid carriers are sugar syrup, peanut oil, olive oil, and water. Examples of gaseous carriers include carbon dioxide and nitrogen.

In preparing the compositions for oral dosage form, any convenient pharmaceutical media may be employed. For example, water, glycols, oils, alcohols, flavoring agents, preservatives, coloring agents, and the like may be used to form oral liquid preparations such as suspensions, elixirs and solutions; while carriers such as starches, sugars, microcrystalline cellulose, diluents, granulating agents, lubricants, binders, disintegrating agents, and the like may be used to form oral solid preparations such as powders, capsules and tablets. Because of their ease of administration, tablets and capsules are the preferred oral dosage units whereby solid pharmaceutical carriers are employed. Optionally, tablets may be coated by standard aqueous or nonaqueous techniques.

A tablet containing the composition of this invention may be prepared by compression or molding, optionally with one or more accessory ingredients or adjuvants. Compressed tablets may be prepared by compressing, in a suitable machine, the active ingredient in a free-flowing form such as powder or granules, optionally mixed with a binder, lubricant, inert diluent, surface active or dispersing agent. Molded tablets may be made by molding in a suitable machine, a mixture of the powdered compound moistened with an inert liquid diluent. Each tablet preferably contains from about 0.05 mg to about 5g of the active ingredient and each cachet or capsule preferably containing from about 0.05mg to about 5g of the active ingredient.

For example, a formulation intended for the oral administration to humans may contain from about 0.5mg to about 5g of active agent, compounded with an appropriate and convenient amount of carrier material which may vary from about 5 to about 95 percent of the total composition. Unit dosage forms will generally contain between from about 1mg to about 2g of the active ingredient, typically 25mg, 50mg, 100mg, 200mg, 300mg, 400mg, 500mg, 600mg, 800mg, or 1000mg.

Pharmaceutical compositions of the present invention suitable for parenteral administration may be prepared as solutions or suspensions of the active compounds in water. A suitable surfactant can be included such as, for example, hydroxypropylcellulose. Dispersions can also be prepared in glycerol, liquid polyethylene glycols, and mixtures thereof in oils. Further, a preservative can be included to prevent the detrimental growth of microorganisms.

Pharmaceutical compositions of the present invention suitable for injectable use include sterile aqueous solutions or dispersions. Furthermore, the compositions can be in the form of sterile powders for the extemporaneous preparation of such sterile injectable solutions or dispersions. In all cases, the final injectable form must be sterile and must be effectively fluid for easy syringability. The pharmaceutical compositions must be stable under the conditions of manufacture and storage; thus, preferably should be preserved against the contaminating action of microorganisms such as bacteria and fungi. The carrier can be a solvent or dispersion medium containing, for example, water, ethanol, polyol (e.g. glycerol, propylene glycol and liquid polyethylene glycol), vegetable oils, and suitable mixtures thereof.

Pharmaceutical compositions of the present invention can be in a form suitable for topical use such as, for example, an aerosol, cream, ointment, lotion, dusting powder, or the like. Further, the compositions can be in a form suitable for use in transdermal devices. These formulations may be prepared, using a compound of formula (I), or a pharmaceutically acceptable salt thereof, via conventional processing methods. As an example, a cream or ointment is prepared by admixing hydrophilic material and water, together with about 5wt% to about 10wt% of the compound, to produce a cream or ointment having a desired consistency.

Pharmaceutical compositions of this invention can be in a form suitable for rectal administration wherein the carrier is a solid. It is preferable that the mixture forms unit dose suppositories. Suitable carriers include cocoa butter and other materials commonly used in the art. The suppositories may be conveniently formed by first admixing the composition with the softened or melted carrier(s) followed by chilling and shaping in molds.

In addition to the aforementioned carrier ingredients, the pharmaceutical formulations described above may include, as appropriate, one or more additional carrier ingredients such as diluents, buffers, flavoring agents, binders, surface-active agents, thickeners, lubricants, preservatives (including anti-oxidants) and the like. Furthermore, other adjuvants can be included to render the formulation isotonic with the blood of the intended recipient. Compositions containing a compound of formula (I), or pharmaceutically acceptable salts thereof, may also be prepared in powder or liquid concentrate form.

Generally, dosage levels on the order of 0.01mg/kg to about 150mg/kg of body weight per day are useful in the treatment of the above-indicated conditions, or alternatively about 0.5mg to about 7g per patient per day. For example, obesity may be effectively treated by the administration of from about 0.01 to 50mg of the compound per kilogram of body weight per day, or alternatively about 0.5mg to about 3.5g per patient per day.

It is understood, however, that the specific dose level for any particular patient will depend upon a variety of factors including the age, body weight, general health, sex, diet, time of administration, route of administration, rate of excretion, drug combination and the severity of the particular disease undergoing therapy.

The compounds of formula (I) may be used in the treatment of diseases or conditions in which GPR119 plays a role.

Thus the invention also provides effective amount of a compound of formula (I), or a pharmaceutically acceptable salt thereof, for use in treating diseases or conditions in which GPR119 plays a role, including obesity and diabetes. In the context of the present application the treatment of obesity is intended to encompass the treatment of diseases or conditions such as obesity and other eating disorders associated with excessive food intake e.g. by reduction of appetite and body weight, maintenance of weight reduction and prevention of rebound and diabetes (including Type 1 and Type 2 diabetes, impaired glucose tolerance, insulin resistance and diabetic complications such as neuropathy, nephropathy, retinopathy, cataracts, cardiovascular complications and dyslipidaemia). And the treatment of patients who have an abnormal sensitivity to ingested fats leading to functional dyspepsia. The compounds of the invention may also be used for treating metabolic diseases such as metabolic syndrome (syndrome X), impaired glucose tolerance, hyperlipidemia, hypertriglyceridemia, hypercholesterolemia, low HDL levels and hypertension.

The invention also provides compounds of formula I for use in the regulation of satiety. The invention also provides compounds of formula I for use in treating obesity.

The invention also provides compounds of formula I for use in the treatment of diabetes, including type 1 and type 2 diabetes, particularly type 2 diabetes.

The invention also provides compounds of formula I for use in the treatment of metabolic syndrome (syndrome X), impaired glucose tolerance, hyperlipidemia, hypertriglyceridemia, hypercholesterolemia, low HDL levels or hypertension.

We also disclose methods of treatment of each of the above conditions, and use of compounds of formula I in the manufacture of a medicament for the above conditions.

In the methods of the invention the term "treatment" includes both therapeutic and prophylactic treatment.

The compounds of formula (I) may exhibit advantageous properties compared to known GPR 119 agonists, for example, the compounds may exhibit improved potency, for example
when compared to compounds wherein group B is unbranched, or improved solubility thus improving absorption properties and bioavailability, or other advantageous properties for compounds to be used as pharmaceuticals.

The compounds of formula (I), or pharmaceutically acceptable salts thereof, may be administered alone or in combination with one or more other therapeutically active compounds. The other therapeutically active compounds may be for the treatment of the same disease or condition as the compounds of formula (I) or a different disease or condition. The therapeutically active compounds may be administered simultaneously, sequentially or separately.

The compounds of formula (I) may be administered with other active compounds for the treatment of obesity and/or diabetes, for example insulin and insulin analogs, gastric lipase inhibitors, pancreatic lipase inhibitors, sulfonyl ureas and analogs, biguanides, α2 agonists, glitazones, PPAR-γ agonists, mixed PPAR-α/γ agonists, RXR agonists, fatty acid oxidation inhibitors, α-glucosidase inhibitors, β-agonists, phosphodiesterase inhibitors, lipid lowering agents, glycogen phosphorylase inhibitors, antiobesity agents e.g. pancreatic lipase inhibitors, MCH-1 antagonists and CB-1 antagonists (or inverse agonists), amylin antagonists, lipoxygenase inhibitors, somostatin analogs, glucokinase activators, glucagon antagonists, insulin signalling agonists, PTP1B inhibitors, gluconeogenesis inhibitors, antilypolitic agents, GSK inhibitors, galanin receptor agonists, anorectic agents, CCK receptor agonists, leptin, serotonergic/dopaminergic antiobesity drugs, reuptake inhibitors e.g. sibutramine, CRF antagonists, CRF binding proteins, thyromimetic compounds, aldose reductase inhibitors, glucocorticoid receptor antagonists, NHE-1 inhibitors or sorbitol dehydrogenase inhibitors.

We also disclose combination therapy comprising the administration of a compound of formula (I), or a pharmaceutically acceptable salt thereof, and at least one other antiobesity agent.

In addition we disclose a method for the treatment of obesity in a mammal, such as a human, which method comprises administering an effective amount of a compound of formula (I), or a pharmaceutically acceptable salt thereof, and another antiobesity agent, to a mammal in need thereof.

We disclose the use of a compound of formula (1), or a pharmaceutically acceptable salt thereof, and another antiobesity agent for the treatment of obesity.

We also disclose the use of a compound of formula (I), or a pharmaceutically acceptable salt thereof, in the manufacture of a medicament for use in combination with another antiobesity agent, for the treatment of obesity.

The compound of formula (I), or a pharmaceutically acceptable salt thereof, and the other antiobesity agent(s) may be co-administered or administered sequentially or separately.

Co-administration includes administration of a formulation which includes both the compound of formula (I), or a pharmaceutically acceptable salt thereof, and the other antiobesity agent(s), or the simultaneous or separate administration of different formulations of each agent. Where the pharmacological profiles of the compound of formula (I), or a pharmaceutically acceptable salt thereof, and the other antiobesity agent(s) allow it, coadministration of the two agents may be preferred.

In addition, we disclose the use of a compound of formula (I), or a pharmaceutically acceptable salt thereof, and another antiobesity agent in the manufacture of a medicament for the treatment of obesity.

We also disclose a pharmaceutical composition comprising a compound of formula (I), or a pharmaceutically acceptable salt thereof, and another antiobesity agent, and a pharmaceutically acceptable carrier. The invention also encompasses the use of such compositions in the methods described above.

GPR119 agonists are of particular use in combination with centrally acting antobesity agents.

The other antiobesity agent for use in the combination therapies disclosed herein is preferably a CB-1 modulator, e.g. a CB-1 antagonist or inverse agonist. Examples of CB-1 modulators include SR141716 (rimonabant) and SLV-319 ((4*S*)-(-)-3-(4-chlorophenyl)-*N-*methyl-*N*-[(4-chlorophenyl)sulfonyl]-4-phenyl-4,5-dihydro-1H-pyrazole-1-carboxamide); as well as those compounds disclosed in EP576357, EP656354, WO 03/018060, WO 03/020217, WO 03/020314, WO 03/026647, WO 03/026648, WO 03/027076, WO 03/040105, WO 03/051850, WO 03/051851, WO 03/053431, WO 03/063781, WO 03/075660, WO 03/077847, WO 03/078413, WO 03/082190, WO 03/082191, WO 03/082833, WO 03/084930, WO 03/084943, WO 03/086288, WO 03/087037, WO 03/088968, WO 04/012671, WO 04/013120, WO 04/026301, WO 04/029204, WO 04/034968, WO 04/035566, WO 04/037823 WO 04/052864, WO 04/058145, WO 04/058255, WO 04/060870, WO 04/060888, WO 04/069837, WO 04/069837, WO 04/072076, WO 04/072077, WO 04/078261 and WO 04/108728, and the references disclosed therein.

Other diseases or conditions in which GPR119 has been suggested to play a role include those described in WO 00/50562 and US 6,468,756, for example cardiovascular disorders, hypertension, respiratory disorders, gestational abnormalities, gastrointestinal disorders, immune disorders, musculoskeletal disorders, depression, phobias, anxiety, mood disorders and Alzheimer's disease.

The invention will now be described by reference to the following examples which are for illustrative purposes and are not to be construed as a limitation of the scope of the present invention.

### EXAMPLES

### Abbreviations.

*t*-Bu: *tert*-Butyl; DCM: Dichloromethane; DMAP: 4-Dimethylaminopyridine; DMF: *N,N-*Dimethylformamide; DMSO: Dimethylsulfoxide; EDCI: 1-(3-Dimethylaminopropyl)-3-ethylcarbodiimide hydrochloride; EtOAc: Ethyl acetate; h: hour; HOBt: 1-Hydroxybenzotriazole hydrate; HPLC: High performance liquid chromatography; *m*CPBA: 3-Chloroperoxybenzoic acid; IH: Isohexane; Me: Methyl; min: Minutes; RP-HPLC: Reverse phase high performance liquid chromatography; TFA: Trifluoroacetic acid; THF: Tetrahydrofuran.

LCMS data were obtained as follows: Waters Atlantis C18, 3µ (3.0 x 20mm, flow rate 0.85 ml/min) eluting with a H₂O-MeCN gradient containing 0.1% v/v HCO₂H over 6.5 min with UV detection at 220nm. Gradient information: 0.0-0.3 min 100% H₂O; 0.3-4.25 min: Ramp to 10% H₂O-90% CH₃CN; 4.25 min-4.4 min: Ramp to 100% CH₃CN; 4.4-4.9 min: Hold at 100% MeCN; 4.9-5.0 min: Return to 100% H₂O; 5.00-6.50 min: Hold at 100% H₂O. The mass spectra were obtained using an electrospray ionisation source in either the positive (ESI⁻) ion or negative ion (ESI⁻) mode. ¹H nmr spectra were recorded on a Varian Mercury 400 spectrometer, operating at 400 MHz. Chemical shifts are reported as ppm relative to tetramethylsilane (δ=0).

HPLC was performed using a Phenomenex™ C₁₈ column (210 x 21mm) eluting with a H₂O-CH₃CN solution at 20mL/min, with UV detection at 220nm. Typical gradient: 0-0.5 min, 10% CH₃CN-90%H₂O; 0.5 min-10 min, ramp to 90% CH₃CN-10% H₂O and hold at 90%CH₃CN-10%H₂O for 5 min; 15 min-16 min, return to 10% CH₃CN-90% H₂O.

3-*tert*-Butyl-5-chloro-[1,2,4]oxadiazole: WO95/05368; 3,5-difluoro-4-methylsulfanylbenzaldehyde: EP1251126; 4-(2-ethoxycarbonyl-1-methylethyl)piperidine-1-carboxylic acid *tert*-butyl ester: WO04/092124; *N*-hydroxy-4-methylsulfanylbenzamidine: Buu-Hoi; Lecocq J, Bull Soc. Chim. Fr. 1946, 139; 1-(3-isopropyl-[1,2,4]oxadiazol-5-yl)piperidin-4-ol: WO05/121121; 4-(2-methoxycarbonylpropyl)piperidine-1-carboxylic acid *tert*-butyl ester: WO98/07620; *N*-Hydroxyisonicotinamidine: Martin A. R. et al, J. Med Chem., 2001, 44, 1560; 2-Methylisonicotinonitrile: Ashimori A. et al, Chem. Pharm. Bull., 1990, 38, 2446.

### Preparation 1: 4-(2-Methoxycarbonylpropyl)piperidine-1-carboxylic acid tert-butyl ester

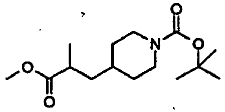

A solution of 4-(2-methoxycarbonylethyl)piperidine-1-carboxylic acid *tert-butyl* ester (780 mg, 2.88 mmol) in anhydrous THF (3 mL) was added dropwise to a stirred solution of lithium diisopropylamide (3.16 mL of a 1M solution in THF, 3.16 mmol) at -78°C under argon. After 1 h the mixture was added slowly *via* cannula to a solution of methyliodide (360 µL, 5.76 mmol) and *N,N*'-dimethylpropyleneurea (1 mL) in anhydrous THF (6 mL) at -78°C. The stirred mixture was warmed to 0°C and, after 4 h, quenched by the addition of saturated aqueous NH₄Cl (5 mL). Ether (100 mL) was added and the organic phase washed with brine (10 mL), dried (MgSO₄) and evaporated to afford an oil that was then purified by column chromatography (IH-EtOAc 7:3) to give the title ester: δ_{H} (EDCl3) 1.01-1.13 (m, 2H), 1.15 (d, 3H), 1.25-1.32 (m, 1H), 1.35-1.44 (m, 1H), 1.45 (s, 9H), 1.58-1.71 (m, 3H), 2.52-2.60 (m, 1H), 2.66 (br t, 2H), 3.68 (s, 3H), 4.01-4.13 (m, 2H).

### Preparation 2: 4-((S)-1-Carboxyethoxy)piperidine-1-carboxylic acid tert-butyl ester

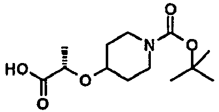

A stirred solution of 4-hydroxypiperidine-1-carboxylic acid *tert*-butyl ester (300 mg, 1.5 mmol) in 1,4-dioxane (4 mL) under an argon atmosphere was treated portionwise with sodium hydride (239 mg of a 60% dispersion in oil, 6 mmol). After 30 min a solution of (*R*)-2-bromopropionic acid (135 µL, 1.5 mmol) in 1,4-dioxane (2 mL) was added and stirring continued for 22 h. Water (15 mL) was added and the aqueous phase washed with ether (15 mL) then acidified to pH 1 using 1M HCl. The suspension was extracted into EtOAc (50 mL), which was dried (MgSO₄) and evaporated to afford the title acid: δ_{H} (CDCl₃) 1.46 (s, 9H), 1.46 (d, 3H), 1.57 (m, 2H), 1.86 (m, 2H), 3.09 (ddd, 2H), 3.61 (sept, 1H), 3.82 (m, 2H), 4.16 (q, 1H).

The acids listed in **Table 1** were prepared using the same method as that described in **Preparation 2:**

**Table 1**

| **Prep** | **Structure** | **Name** | δ**_{H} (CDCl₃)** |
|---|---|---|---|
| **3** | 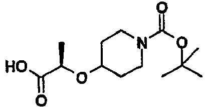 | 4-((*R*)-1-Carboxyethoxy) piperidine-1-carboxylic acid *tert*-butyl ester | 1.47 (s, 9H), 1.47 (d, 3H), 1.57 (m, 2H), 1.86 (m, 2H), 3.08 (ddd, 2H), 3.62 (sept (1H), 3.81 (m, 2H), 4.16 (q, 1H) |
| **4** | 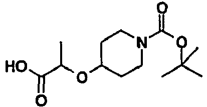 | 4-(1-Carboxyethoxy) piperidine-1-carboxylic acid *tert*-butyl ester | 1.46 (s, 9H), 1.46 (d, 3H), 1.57 (m, 2H), 1.86 (m, 2H), 3.08 (ddd, 2H), 3.59 (sept, 1H), 3.81 (m, 2H), 4.14 (q, 1H) |
| **5** | 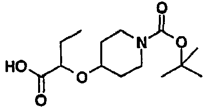 | 4-(1-Carboxypropoxy) piperidine-1-carboxylic acid *tert*-butyl ester | 1.01 (t, 3H), 1.46 (s, 9H), 1.58 (m, 2H), 1.74-1.81 (m, 4H), 3.11 (ddd, 2H), 3.56 (sept, 1H), 3.78 (m, 2H), 3.95 (dd, 1H) |
| **6** | 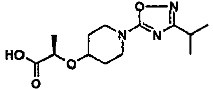 | (*R*)-2-[1-(3-Isopropyl-[1,2,4]oxadiazol-5-yl) piperidin-4-yloxy] propionic acid | 1.29 (d, 6H), 1.48 (d, 3H), 1.75 (m, 2H), 1.94 (m, 2H), 2.90 (sept, 1H), 3.45 (ddd, 2H), 3.7 1 (sept, 1H), 3.87 (ddd, 2H), 4.16 (q, 1H) |
| **7** | 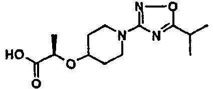 | (*R*)-2-[1-(5-Isopropyl-[1,2,4]oxadiazol-3-yl) piperidin-4-yloxy] propionic acid | 1.35 (d, 6H), 1.49 (d, 3H), 1.71 (m, 2H), 1.96 (m, 2H), 3.08 (sept, 1H), 3.18 (ddd, 2H), 3.68 (sept, 1H), 3.81 (m, 2H), 4.19 (q, 1H) |

### Preparation 8: 3,5-Difluoro-4-methylsulfanylbenzonitrile

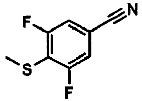

A stirred solution of 3,5-difluoro-4-methylsulfanylbenzaldehyde (1.0 g, 5.32 mmol) in ethanol (12 mL) was treated with NH₂OH.HCl (778 mg, 112 mmol) in water (5 mL) followed immediately by K₂CO₃ (780 mg, 5.64 mmol) in water (10 mL). After 1 h at room temperature the ethanol was largely removed *in vacuo* and water (30 mL) added. The mixture was extracted into EtOAc (150 mL), the organic phase separated, dried (MgSO₄) and evaporated. The residue was purified by column chromatography (IH-EtOAc-CH₂Cl₂ 88:10:2) to afford 3,5-difluoro-4-methylsulfanylbenzaldehyde oxime: δ_{H} (CDCl₃) 2.50 (s, 3H), 7.14 (d, 2H), 7.47 (s, 1H), 8.03 (s, 1H). Acetic anhydride (45 mL) was added to a sample of this oxime (1.139 g, 5.6 mmol) followed by *p*-toluenesulfonic acid monohydrate (1.066 g, 5.6 mmol). The mixture was heated under reflux for 20 h, cooled and the excess acetic anhydride removed *in vacuo.* Water (50 mL) was added and adjusted to pH 8 using saturated aqueous Na₂CO₃, then extracted with CH₂Cl₂ (100 mL). The solvent was removed and the residue purified by column chromatography (IH-EtOAc 9:1) to give the title nitrile: δ_{H} (CDCl₃) 2.58 (s, 3H), 7.20 (d, 2H).

### Preparation 9: 3-Fluoro-4-methylsulfanylbenzonitrile

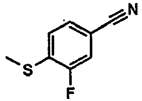

Sodium thiomethoxide (1.59 g, 22.6 mmol) was suspended in anhydrous DMF (35 mL) and cooled to 5°C. A solution of 3,4-difluorobenzonitrile (3.0 g, 21.6 mmol) dissolved in cold DMF (25 mL) was added *via* cannula and the mixture stirred for 1 h. The DMF was evaporated and the residual solid recrystallised from ether triturated with hexane, giving the title nitrile: δ_{H} (CDCl₃) 2.53 (s, 3H), 7.26 (t, 1H), 7.29 (dd, 1H), 7.43 (dd, 1H).

### Preparation 10: 3-Methyl-4-methylsulfanylbenzonitrile

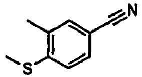

Using the procedure outlined in **Preparation 9,** 4-fluoro-3-methylbenzonitrile was converted to the title compound: δ_{H} (CDCl₃) 2.33 (3H, s), 2.52 (3H, s), 7.16 (1H, d), 7.35 (1H, s) 7.47 (1H, d).

### Preparation 11: 4-Hydroxypiperidine-1-carbonitrile

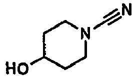

A stirred slurry of Na₂CO₃ (9.8 g, 117 mmol) in water (15 mL) was cooled on ice-water and 4-hydroxypiperidine.HCl (4.0 g, 29.2 mmol) was added. A solution of cyanogen bromide (3.7 g, 35 mmol) in CH₂Cl₂ (90 mL) was added portionwise over 5 min and the stirring continued for 1 h. The reaction was diluted with CH₂Cl₂ (100 mL), the organic phase separated and dried (MgSO₄) and the solvent evaporated to afford the title cyanamide: δ_{H} (CDCl₃) 1.65 (ddt, 2H), 1.91 (ddt, 2H), 2.17 (br s, 1H), 3.08 (ddd, 2H), 3.45 (ddd, 2H), 3.86 (sept, 1H).

### Preparation 12: 4-Cyano-2-fluorobenzenesulfonamide

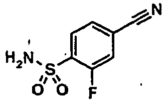

3-Fluoro-4-methylsulfanylbenzonitrile (1.5 g, 8.9 mmol) was dissolved in CH₂Cl₂ (75 mL) and water (15 mL) added. Chlorine gas was bubbled gently through the vigorously-stirred mixture for 30 min and the organic component then separated and evaporated to dryness. The residue was dissolved in thionyl chloride (30 mL) and heated under reflux for 6.5 h. The mixture was evaporated to dryness to afford near-pure 4-cyano-2-fluorobenzenesulfonyl chloride: δ_{H} (EDCl₃) 7.67 (d, 1H), 7.70 (d, 1H), 8.14 (dd, 1H). A sample of the sulfonyl chloride (1.53 g, 7 mmol) was dissolved in CH₂Cl₂ and ammonia was bubbled gently through the stirred solution. After 10 min the mixture was filtered, the filtrate evaporated and the residue purified by column chromatography (IH-EtOAc 2:1) to give the title sulfonamide: δ_{H} (d₆ DMSO) 7.85 (dd, 1H), 7.96 (t, 1H), 7.96 (br s, 2H), 8.12 (dd, 1H).

### Preparation 13: 3-Fluoro-N-hydroxy-4-sulfamoylbenzamidine

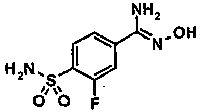

Water (10 mL) was added to a mixture of K₂CO₃ (491 mg, 3.55 mmol) and NH₂OH.HCl (492 mg, 7.1 mmol) and the resulting solution added to a stirred solution of 4-cyano-2-fluorobenzenesulfonamide (710 mg, 3.55 mmol) in ethanol (60 mL). The mixture was heated under reflux for 18 h, cooled and partitioned between EtOAc (250 mL) and water (100 mL). The organic phase was separated, dried (MgSO₄) and evaporated and the residue purified by column chromatography (EtOAc) to afford the title compound (d₆, DMSO) 6.00 (s, 2H), 7.63 (dd, 1H), 7.66 (dd, 1H), 7.67 (s, 2H), 7.77 (t, 1H), 10.01 (s, 1H).

The amidoximes listed in **Table 2** were prepared using a similar method to that of **Preparation 13**.

**Table 2**

| **Prep** | **Structure** | **Name** | **LCMS data /¹H nmr data** |
|---|---|---|---|
| **14** | 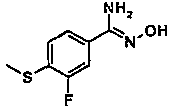 | 3-Fluoro-*N*-hydroxy-4-methylsulfanylbenzamidine | δ_{H} (CDCl₃) 2.50 (s, 3H), 4.84 (s, 2H), 7.26 (t, 1H), 7.32 (d, 1H), 7.38 (dd, 1H), 7.75 (s, 1H) |
| **15** | 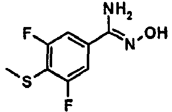 | 3,5-Difluoro-*N*-hydroxy-4-methylsulfanylbenzamidine | δ_{H} (CDCl₃) 2.50 (s, 3H), 4.79 (s, 2H), 6.64 (s, 1H), 7.20 (d, 2H) |
| **16** | 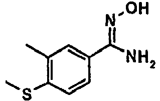 | *N*-Hydroxy-3-methyl-4-methylsulfanylbenzamidine | δ_{H} (CDCl₃) 2.35 (3H, s), 2.50 (3H, s), 4.83 (2H, s), 6.83 (1H, s), 7.16 (1H, d), 7.42 (1H, s), 7.45 (1H, d) |
| **17** | 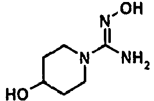 | 4-*N*-Dihydroxypiperidine-1-carboxamidine | RT = 0.28 min; *m*/*z* (ES⁻) = 160.0 [M+H] |
| **18** | 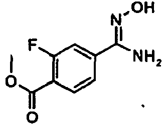 | 2-Fluoro-4-(*N*-hydroxy-carbamimidoyl)benzoic acid methyl ester | δ_{H} (CD₃OD) 3.91 (3H, s), 7.50 (1H, dd), 7.56 (1H, dd), 7.93 (1H, t) |
| **19** | 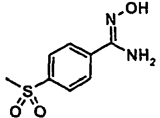 | *N*-Hydroxy-4-methane-sulfonylbenzamidine | δ_{H} (d₆ DMSO) 3.22 (3H, s), 5.99 (2H, s), 7.92 (4H, s), 9.97 (1H, s) |
| **20** | 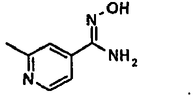 | *N*-Hydroxy-2-methylisonicotinamidine | δ_{H} (d₆ DMSO) 2.47 (3H, s), 5.93 (2H, s), 7.43 (1H, d), 7.5 1 (1H, s), 8.42 (1H, d), 9.96 (1H, s) |

### Preparation 21: 4-(1-Carboxyethoxy)piperidine-1-carboxylic acid tert-butyl ester

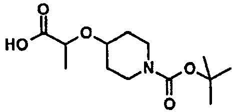

A stirred solution of 4-hydroxypiperidine-1-carboxylic acid *tert*-butyl ester (2 g, 9.94 mmol) in anhydrous dioxane (20 mL) was treated portionwise with NaH (828 mg of a 60% dispersion in oil, 20 mmol). The temperature was raised to 65°C and, after 5 min, a solution of 2-bromopropionic acid (0.89 mL, 9.94 mmol) in dry dioxane (20 mL) was added in a dropwise manner. After 3 h at this temperature further NaH (828 mg) was added and heating continued for 18 h. The cooled reaction was quenched by the careful addition of water (50 mL) the mixture was washed with EtOAc (50 mL). This organic layer was extracted with 1M aqueous NaOH (20 mL) and the combined aqueous phases acidified to pH 2 using conc HCl. After extraction with EtOAc (3 × 50 mL) the combined organic extracts were dried (MgSO₄) and evaporated to afford the title compound: δ_{H} (CDCl₃) 1.46 (9H, s), 1.48 (3H, s), 1.57 (2H, m), 1.86 (2H, m), 3.09 (2H, ddd), 3.61 (1H, tt), 3.80 (2H, m), 4.15 (1H, q).

### Preparation 22: 4-Cyano-2-fluorobenzoic acid methyl ester

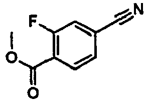

Sufficient methanol (ca. 10 mL) was added to a stirred suspension of 4-cyano-2-fluorobenzoic acid (2 g, 12.11 mmol) in toluene (5 mL) to give a clear solution. Trimethylsilyldiazomethane (7.87 mL of a 2 M solution in hexane, 15.75 mmol) was added dropwise, until the persistence of a yellow colour, after which the solution was stirred for a further 10 min and AcOH then added until a colourless solution was again obtained. The reaction mixture was diluted with EtOAc (50 mL) and washed with saturated aqueous Na₂CO₃ (2 x 20 mL) and brine (20 mL) and dried (MgSO₄). Evaporation of the solvent afforded the title ester: δ_{H} (CDCl₃) 3.98 (3H, s), 7.47 (1H, dd), 7.53 (1H, dd), 8.06 (1H, t).

### Preparation 23: 1-(5-Isopropyl[1,2,4]oxadiazol-3-yl)piperidin-4-ol

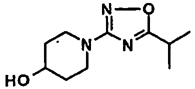

Isobutyric acid (360 µL, 3.9 mmol), 4-*N*-dihydroxypiperidine-1-carboxamidine (620 mg, 3.9 mmol) and HOBt (656 mg, 4.3 mmol) were dissolved in dry DMF (5 mL). Diisopropylethylamine (2.2 mL, 12.9 mmol) was added followed by EDCI (900 mg, 4.7 mmol) in one portion and the mixture stirred for 18 h. Water (20 mL) was added and the mixture extracted with EtOAc (150 mL). The organic phase was washed with water (15 mL) saturated aqueous Na₂CO₃ (15 mL) and brine (10 mL) then dried (MgSO₄) and evaporated to give the title oxadiazole: RT = 2.45 min; *m*/*z*(ES⁺) = 212.0 [*M*+H]⁺.

### Example 1: 4-{2-[3-(3-Fluoro-4-methylsulfanylphenyl)-[1,2,4]oxadiazol-5-yl]-1-methylethyl}piperidine-1-carboxylic acid tert-butyl ester

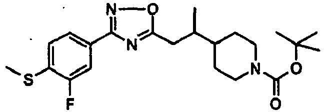

A stirred solution of 3-fluoro-N-hydroxy-4-methylsulfanylbenzamidine (200 mg, 1 mmol) in anhydrous THF (10 mL) was treated with sodium hydride (33.3 mg, 832 µmol). After 40 min a solution of 4-(2-ethoxycarbonyl-1-methylethyl)piperidine-1-carboxylic acid *tert*-butyl ester (249 mg, 832 µmol) in dry THF (3 mL) was added and the mixture heated under reflux for 18 h. The solvent was removed and the residue purified by column chromatography (IH-EtOAc 4:1) to afford the title oxadiazole: δ_{H} (CDCl₃) 0.99 (d, 3H), 1.22-1.31 (m, 2H), 1.39-1.45 (m, 1H), 1.47 (s, 9H), 1.68 (br d, 2H), 2.08 (m, 1H), 2.53 (s, 3H), 2.66 (m, 2H), 2.80 (dd, 1H), 3.00 (dd, 1H), 4.18 (m, 2H), 7.31 (t, 1H), 7.74 (dd, 1H), 7.84 (dd, 1H).

### Example 2: 4-{2-[3-(3-Fluoro-4-methylsulfanylphenyl)-[1,2,4]oxadiazol-5-yl]propyl}-piperidine-1-carboxylic acid tert-butyl ester

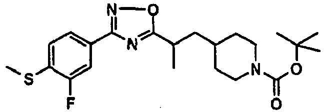

Using a similar procedure to that in **Example 1,** 3-fluoro-*N*-hydroxy-4-methylsulfanylbenzamidine was reacted with 4-(2-methoxycarbonylpropyl)piperidine-1-carboxylic acid *tert*-butyl ester to give the title oxadiazole: RT = 4.45 min; *m*/*z* (ES⁺) = 436.1 [M+H]⁻.

### Example 3: 4-{1-[3-(3-Fluoro-4-methylsulfanylphenyl)-[1,2,4]oxadiazol-5-yl]propoxy}-piperidine-1-carboxylic acid tert-butyl ester

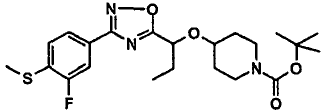

4-(1-Carboxypropoxy)piperidine-1-carboxylic acid *ter*t-butyl ester (167 mg, 580 µmol), EDCI (111 mg, 580 µmol) and HOBt (78 mg, 580 µmol) were dissolved in dry THF (10 mL), diisopropylethylamine (300 µL, 1.75 mmol) added and the mixture stirred for 15 min. Solid 3-fluoro-*N*-hydroxy-4-methylsulfanylbenzamidine (106 mg, 530 mol) was added in one portion and stirring continued for 4 h. The reaction mixture was diluted with EtOAc (50 mL), washed with saturated aqueous Na₂CO₃ (10 mL) and brine (10 mL) then dried (MgSO₄) and evaporated. The residue was taken up in toluene (15 mL), the solution heated under reflux for 18 h. Removal of the solvent and purification of the residue by column chromatography (IH-EtOAc 4:1) afforded the title oxadiazole: RT = 4.42 min; *m*/*z* (ES⁻) = 452.1 [M+H]⁻.

The compounds listed in **Table 3** were synthesised using the same procedure as that outlined in **Example 3**.

**Table 3**

| **Ex** | **Structure** | **Name** | **LCMS data** |
|---|---|---|---|
| **4** | 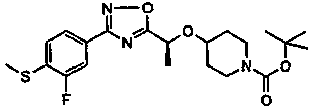 | 4-{(*S*)-1-[3-(3-Fluoro-4-methylsulfanylphenyl)-[1,2,4]oxadiazol-5-yl] ethoxy}piperidine-1-carboxylic acid *tert*-butyl ester | RT = 4.29 min *m*/*z* (ES⁻) = 438.0 [*M*+H]⁺ |
| **5** | 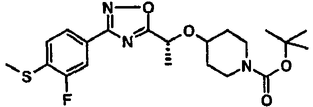 | 4-{(*R*)-1-[3-(3-Fluoro-4-methylsulfanylphenyl)-[1,2,4]oxadiazol-5-yl] ethoxy}piperidine-1-carboxylic acid *tert*-butyl ester | RT = 4.36 min *m*/*z* (ES⁺) = 438.0 [*M*+H]⁺ |
| **6** | 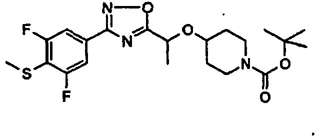 | 4-{1-[3-(3,5-Difluoro-4-methylsulfanylphenyl)-[1,2,4]oxadiazol-5-yl]-ethoxy}piperidine-1-carboxylic acid *tert*-butyl ester | RT = 4.44 min *m*/*z* (ES⁻) = 456.0 [*M*+H] |
| **7** | 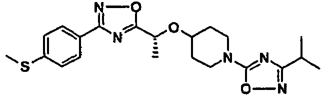 | 1-(3-Isopropyl-[1,2,4] oxadiazol-5-yl)-4-{(*R*)-1-[3-(4-methylsulfanyl phenyl)-[1,2,4]oxadiazol-5-yl]ethoxy}piperidine | RT=4.11 min *m*/*z* (ES⁺) = 430.0 [*M*+H]⁻ |
| **8** | 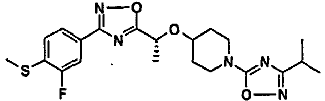 | 4-{(*R*)-1-[3-(3-Fluoro-4-methylsulfanylphenyl)-[1,2,4]oxadiazol-5-yl] ethoxy} -1-(3-isopropyl-[1,2,4]oxadiazol-5-yl)-piperidine | RT = 4.20 min *m*/*z* (ES⁻) = 448.0 [*M*+H]⁻ |
| **9** | 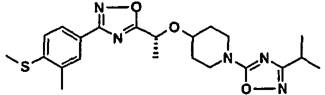 | 1-(3-Isopropyl-[1,2,4] oxadiazol-5-yl)-4-{(*R*)-1-[3-(3-methyl-4-methyl sulfanylphenyl)-[1 ,2,4] oxadiazol-5-yl]ethoxy}piperidine | RT=4.53 min *m*/*z* (ES⁻) = 444.2 [*M*+H]⁻ |
| **10** | 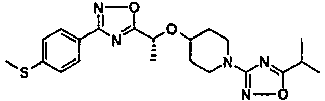 | 1-(5-Isopropyl-[1,2,4] oxadiazol-3-yl)-4-{(*R*)-1-[3-(4-methylsulfanyl phenyl)-[1,2,4]oxadiazol-5-yl]ethoxy}piperidine | RT = 4.53 min *m*/*z* (ES⁺) - 430.3 [*M*+H]⁺ |
| **11** | 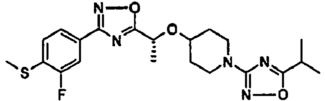 | 4-{(*R*)-1-[3-(3-Fluoro-4-methylsulfanylphenyL)-[1,2,4]oxadiazol-5-yl] ethoxyl}-1-(5-isopropyl-[1,2,4]oxadiazol-3-yl) piperidine | RT = 4.62 min *m*/*z* (ES⁺) = 448.2 [*M*+H]⁺ |
| **12** | 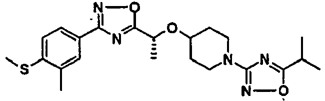 | 1-(5-Isopropyl-[1,2,4] oxadiazol-3-yl)-4-{(*R*)-1-[3-(3-methyl-4-methyl sulfanylphenyl)-[1,2,4] oxadiazol-5-yl]ethoxy} pipelidine | RT = 4.71 min *m*/*z* (ES⁺) = 444.3 [*M*+H]⁻ |
| **13** | 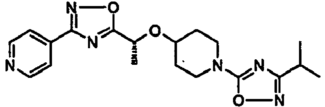 | 4-(5-{(*R*)-1-[1-(3-Isopropyl-[1,2,4] oxadiazol-5-yl)piperidin-4-yloxy]ethyl}-[1,2,4] oxadiazol-3 -yl)pyridine | RT = 3.34 min *m*/*z* (ES⁺) = 384.9 [*M*+H]⁺ |
| **14** | 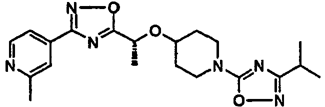 | 4-(5-{(*R*)-1-[1-(3-Isopropyl-[1,2,4] oxadiazol-5-yl)piperidin-4-yloxy]ethyl}-[1,2,4] oxadiazol-3 -yl)-2-methyl-pyridine | RT= 3.15 min *m*/*z* (ES⁺) = 398.9 [*M*+H]⁺ |
| **15** | 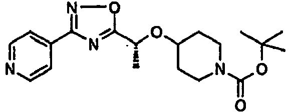 | 4-[(*R*)-1-(3-Pyridin-4-yl-[1,2,4]oxadiazol-5-yl) ethoxy]piperidine-1-carboxylic acid *tert*-butyl ester | RT = 3.49 min *m*/*z* (ES⁺) = 375.0 [*M*+H]⁻ |
| **16** | 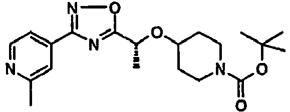 | 4-{(*R*)-1-[3-(2-Methyl pyridin-4-yl)-[1,2,4] oxadiazol-5-yl]ethoxy} piperidine-1-carboxylic acid *tert*-butyl ester | RT = 3.39 min *m*/*z* (ES⁻) = 389.0 [*M*+H]⁺ |

### Example 17: 1-(3-tert-Butyl-[1,3,4]oxadiazol-5-yl)-4-{(R)-1-[3-(3-fluoro-4-methylsulfanylphenyl)-[1,2,4]oxadiazol-5-yl]ethoxy}piperidine

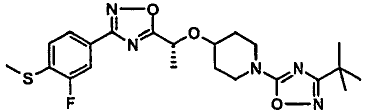

4- {(*R*)-1-[3-(3-Fluoro-4-methylsulfanylphenyl)-[1,2,4]oxadiazol-5-yl]ethoxy}-piperidine-1-carboxylic acid *tert*-butyl ester (133 mg, 304 µmol) was dissolved in 4M HCl in dioxane (10 mL). After stirring for 30 min the solvent was removed to afford the HCl salt of 4-{(*R*)-1-[3-(3-fluoro-4-methylsulfanylphenyl)-[1,2,4]oxadiazol-5-yl]ethoxy}piperidine: RT = 2.69 min; *m*/*z* (ES⁺) = 338.0 [*M*+H]⁻. A sample of this salt (114 mg, 304 µmol) and 3-*tert*-butyl-5-chloro-[1,2,4]oxadiazole (73 mg, 456 µmol) were dissolved in anhydrous DMF (10 mL) and K₂CO₃ (63 mg, 456 µmol) added. After stirring for 18 h the solvent was removed and the residue dissolved in EtOAc (20 mL). The organic phase was washed with 2M NaOH (5 mL) and brine and dried (MgSO₄). Removal of the solvent afforded the title oxadiazole: RT = 4.29 min; *m*/*z* (ES⁺) = 462.1 [*M*+H]⁺.

### Example 18: 4-{1-[3-(3-Fluoro-4-methanesulfinylphenyl)-[1,2,4]oxadiazol-5-yl]propoxy}-piperidine-1-carboxylic acid tert-butyl ester

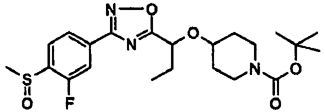

Solid *m*CPBA (69 mg of 78% w/w, 310 µmol) was added to a stirred solution of 4-{1-[3-(3-fluoro-4-methylsulfanylphenyl)-[1,2,4]oxadiazol-5-yl]propoxy; piperidine-1-carboxylic acid *tert*-butyl ester (140 mg, 310 µmol) in CH₂Cl₂ (5 mL). After 1 h the solvent was removed and EtOAc (10 mL) added. This solution was washed with saturated aqueous Na₂CO₃ (5 mL), water (5 mL) and brine (5 mL) then dried (MgSO₄) and evaporated. The residue was purified by column chromatography (1H-EtOAc 1:1 the 1:2) to afford the title sulfoxide: RT = 4.42 min; *m*/*z* (ES⁻) = 452.1 [M+H]⁺. Using two equivalents of *m*CPBA affords the corresponding sulfone.

The compounds in **Table 4** were produced by oxidising the corresponding sulfide using the method of **Example 18**.

**Table 4**

| **Ex** | **Structure** | **Name** | **LCMS data / ¹H nmr data** |
|---|---|---|---|
| **19** | 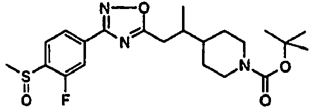 | 4-{2-[3-(3-Fluoro-4-methane sulfinylphenyl)-[1,2,4]oxadiazol-5-yl]-1-methylethyl}-piperidine-1-carboxylic acid *tert*-butyl ester | RT = 3.91 min *m*/*z* (ES⁻) = 452.0 [*M*+H]⁺ |
| **20** | 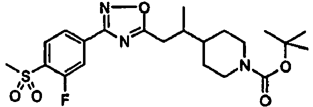 | 4-{2-[3-(3-Fluoro-4-methane sulfonylphenyl)-[1,2,4]oxadiazol-5-yl]-1-methylethyl}-piperidine-1-carboxylic acid *tert*-butyl ester | RT = 4.02 min *mlz* (ES⁻) = 485.1 [*M*+NH₄]⁻ |
| **21** | 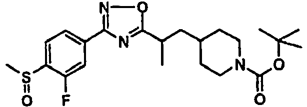 | 4-{2-[3-(3-Fluoro-4-methane sulfinylphenyl)-[1,2,4]oxadiazol-5-yl]propyl}piperidine-1-carboxylic acid *tert-*butyl ester | RT = 3.94 min *m*/*z* (ES⁻) = 452.1 [*M*+H]⁺ |
| **22** | 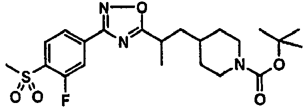 | 4-{2-[3-(3-Fluoro-4-methane sulfonylphenyl)-[1,2,4]oxadiazol-5-yl]propyl}piperidine-1-carboxylic acid *tert*-butyl ester | RT = 4.07 min *m*/*z* (ES⁻) = 485.1 [*M*+NH₄]⁺ |
| **23** | 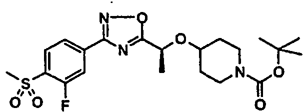 | 4-{(*S*)-1-[3-(3-Fluoro-4-methane sulfonylphenyl)-[ 1,2,4] oxadiazol-5-yl]ethoxy} piperidine-1-carboxylic acid *tert*-butyl ester | RT = 3.84 min *m*/*z* (ES⁻) = 470.1 [*M*+H]⁻ |
| **24** | 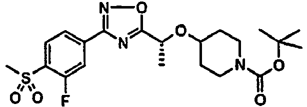 | 4-{(*R*)-1-[3-(3-Fluoro-4-methanesulfonyl phenol)-[1,2,4] oxadiazol-5-yl] etboxy}pipendine-1-carboxylic acid *tert*-butyl ester | RT = 3.82 min; *m*/*z* (ES⁻) = 470.1 [*M*+H]⁻; δ_{H} (CDCl₃) 1.46 (s, 9H), 1.60 (m, 2H), 1.68 (d, 3H), 1.79 (m, 1H), 1.90 (m, 1H), 3.13 (dddd, 2H), 3.28 (s, 3H), 3.67 (sept, 1H), 3.77 (m, 2H), 4.95 (q, 1H), 8.00 (d, 1H), 8.08-8.13 (m, 2H) |
| **25** | 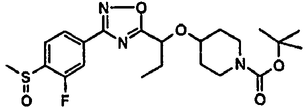 | 4-{1-[3-(3-Fluoro-4-methanesulfinyl phenyl)-[1,2,4] oxadiazol-5-yl] propoxy}piperidine-1-carboxylic acid *tert*-butyl ester | RT = 3.79 min *m*/*z* (ES⁻) = 468.0 [*M*+H]⁻ |
| **26** | 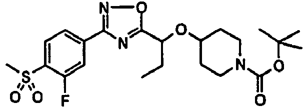 | 4-{1-[3-(3-Fluoro-4-methanesulfonyl phenyl)-[1,2,4] oxadiazol-5-yl] propoxy}piperidine-1-carboxylic acid *tert-*butyl ester | RT = 3.94 min *m*/*z* (ES⁻) = 484.0 [*M*+H]⁻ |
| **27** | 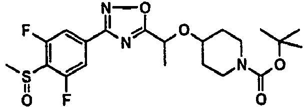 | 4-{1-[3-(3.5-Ditluoro-4-methanesulfinyl phenyl)-[1,2,4] oxadiazol-5-yl]ethoxy} piperidine-1-carboxylic acid *tert*-butyl ester | RT = 3.62 min *m*/*z* (ES⁻) = 472.0 [*M*+H]⁻ |
| **28** | 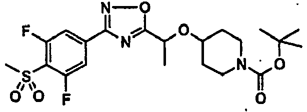 | 4- {1-[3-(3,5-Difluoro-4-methanesulfonyl phenyl)-[1,2,4] oxadiazol-5-yl]ethoxy} piperidine-1-carboxylic acid *tert*-butyl ester | RT = 3.76 min *m*/*z* (ES⁺) = 488.0 [*M*+H]⁺ |
| **29** | 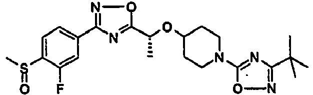 | 1-(3-*tert*-Butyl-[1,2,4] oxadiazol-5-yl)-4-{(*R*)-1-[3-(3-fluoro-4-methanesulfinylphenyl) -[1,2,4]oxadiazol-5-yl]-ethoxy}piperidine | RT = 3.70 min *m*/*z* (ES⁺) = 478.2 [M+H]⁺ |
| **30** | 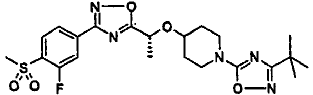 | 1-(3-*tert*-Buty)-[1,2,4] oxadiazol-5-yl)-4-{(*R*)-1-[3-(3-fluoro-4-methanesulfonylphenyl) -[1,2,4]oxadiazol-5-yl]ethoxy} piperidine | RT = 3.96 min *m*/*z* (ES⁺) = 494.2 [*M*+H]⁻ |
| **31** | 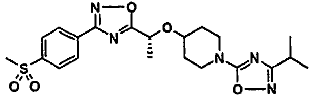 | 1-(3-Isopropyl-[1,2,4] oxadiazol-5-yl)-4-{(*R*)-1-[3-(4-methane sulfonylphenyl)-[1,2,4] oxadiazol-5-yl]ethoxy} piperidine | RT=3.62min *m*/*z* (ES⁻) = 462.0 **[*M*+H]⁻** |
| **32** | 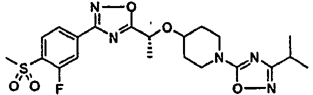 | 4-{(*R*)-1-[3-(3-Fluoro-4-methanesulfonyl phenyl)-[1,2,4] oxadiazol-5-yl]ethoxy}-1-(3-isopropyl-[1,2,4] oxadiazol-5-yl) piperidine | RT=3.69 min; *m*/*z* (ES⁻) = 480.0 [*M*+H]⁻; δ_{H} (CDCl₃) 1.29 (d, 6H), 1.71 (d, 3H), 1.79 (m, 2H), 1.90 (m, 1H), 2.00 (m, 1H), 2.89 (sept, 1H), 3.28 (s, 3H), 3.48 (ddt, 2H), 3.77-3.89 (m, 3H), 4.97 (q, 1H), 8.01 (d, 1H), 8.06-8.14 (m, 2H) |
| **33** | 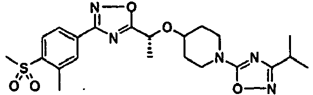 | 1-(3-Isopropyl-[1,2,4] oxadiazol-5-yl)-4-{(*R*)-1-[3-<4-methane sulfonyl-3-methyl | RT = 3.70 min; *m*/*z* (ES⁻) = 476.0 [M+H]⁻; δ_{H} (CDCl₃) 1.29 (d, 6H), 1,71 (d, 3H), 1.78 (m, 2H), 1.89 (m, 1H), 1.99 (m, 1H), 2.81 (s, |
| | | phenyl)-[1,2,4] oxadiazol-5-yl] ethoxy}piperidine | 3H), 2.89 (sept, 1H), 3.13 (s, 3H), 3.49 (ddt, 2H), 3.76-3.89 (m, 3H), 4.97 (q, 1H), 8.10-8.12 (m, 2H), 8.18 (d, 1H) |
| **34** | 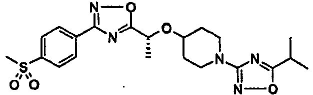 | 1-(5-Isopropyl-[1,2,4] oxadiazol-3-yl)-4-{(*R*)-1-[3-(4-methane sulfonylphenyl)-[1,2,4]oxadiazol-5-yl] ethoxy}piperidine | RT = 3.69 min *m*/*z* (ES⁺) = 462.0 [*M*+H]⁺ |
| **35** | 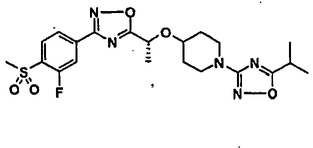 | 4-{(*R*)-1-[3-(3-Fluoro-4-methanesul fonyl phenyl)-[1,2,4] oxadiazol-5-yl]ethoxy}-1-(5-isopropyl-[1,2,4] oxadiazol-3-yl) piperidine | RT = 3.84 min; *m*/*z* (ES⁺) = 480.0 [*M*+H]⁺; δ_{H} (CDCl₃) 1.34 (d, 6H), 1.69 (d, 3H), 1.74 (m, 2H), 1.89 (m, 1H), 2.01 (m, 1H), 3.06 (sept, 1H), 3.22 (dddd, 2H), 3.28 (s, 3H), 3.70-3.81 (m, 3H), 4.97 (q, 1H), 8.00 (d, 1H), 8.07-8.12 (m, 2H) |
| **36** | 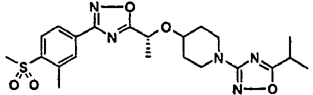 oxadiazol-5-yl]ethoxyl | 1-(5-Isopropyl-[1,2,4] oxadiazol-3-yl)-4-{(*R*)-1-[3-(4-methane sulfonyl-3-methyl phenyl)-[1,2,4] oxadiazol-5-yl]ethoxyl piperidine | RT = 3.89 min; *m*/*z* (ES⁺) = 476.0 [*M*+H]⁺; δ_{H} (CDCl₃) 1.35 (d, 6H), 1.70 (d, 3H), 1.75 (m, 2H), 1.89 (m, 1H), 2.00 (m, 1H), 2.81 (s, 3H), 3.07 (sept, 1H), 3.13 (s, 3H), 3.22 (dddd, 2H), 3.70-3.82 (m, 3H), 4.98(q, 1H), 8.11-8.13(m, 2H), 8.18 (d, 1H) |

The compounds listed in **Table 5** were synthesised by reaction of 3-fluoro-*N*-hydroxy-4-sulfamoylbenzamidine with the corresponding acid and cyclising to the oxadiazole using the method described in **Example 18**.

**Table 5**

| **Ex** | **Structure** | **Name** | **LCMS data** |
|---|---|---|---|
| **37** | 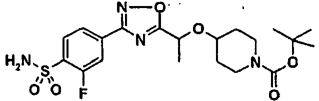 | 4-{1-[3-(3-Fluoro-4-sulfamoylphenyl)-[1,2,4]oxadiazol-5-yl] ethoxy}piperidine-1-carboxylic acid *tert-*butyl ester | RT=3.54min *m*/*z* (*ES⁺*) = 471.0 [*M*+H]⁺ |
| **38** | 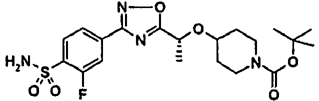 | 4-{(*R*)-1-[3-(3-Fluoro-4-sulfamoylphenyl)-[1.2,4]oxadiazol-5-yl] ethoxy} piperidine-1-carboxylic acid *tert-*butyl ester | RT = 3.62 min *m*/*z* (ES⁻) = 471.1 [*M*+H]⁻ |

### Example 39: 4-{(R)-1-[3-(3-Fluoro-4-methanesulfonylphenyl)-[1,2,4]oxadiazol-5-yl]ethoxy}piperidine-1-carboxylic acid isopropyl ester

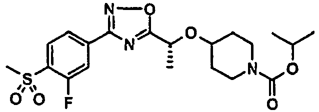

4- {(*R*)-1-[3-(3-Fluoro-4-methanesulfonylphenyl)-[1,2,4]oxadiazol-5-yl]ethoxy}piperidine-1-carboxylic acid *tert*-butyl ester (89 mg, 190µmol) was dissolved in 4M HCl in dioxane (5 mL) and stirred for 1 h. The solvent was removed to afford the HCl salt of 4-{(*R*)-1-[3-(3-fluoro-4-methanesulfonylphenyl)-[1,2,4]oxadiazol-5-yl]ethoxy)piperidine: RT= 2.20 min; *m*/*z* (ES⁻) = 370.0 [*M* + H]⁺. This material was dissolved in CH₂Cl₂ (15 mL) and triethylamine, (65 µL, 460 µmol) and isopropylchloroformate (239 µmol, of a 1M solution in CH₂Cl₂, 239 mmol) added. After stirring for 30 min the solvent was removed and the residue purified by column chromatography (1H-EtOAc 7:3) to give the title carbamate: RT = 3.70 min; *m*/*z* (ES⁺) = 456.0 [*M*+H]⁻.

### Example 40: 4-{1-[3-(4-Methanesulfonylphenyl)-[1,2,4]oxadiazol-5-yl]ethoxy}piperidine-1-carboxylic acid tert-butyl ester

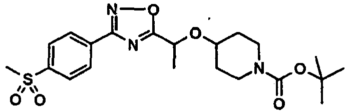

EDC (107 mg, 560 µmol) and HOBt (76 mg, 560 ,umol) were added quickly to a solution of 4-(1-carboxyettioxy)pipeiidine-1-carboxylic acid *tert*-butyl ester (153 mg, 56 µmol) in anhydrous THF. The resulting mixture was stirred under argon for 10 min then solid *N-*hydroxy-4-methanesulfonylbenzamidine was added in one portion and the stirring continued for 18 h. The reaction mixture was diluted with EtOAc (20 mL) and washed with 2M aqueous HCl (2 × 10 mL) and saturated aqueous Na₂CO₃ (3 × 10 mL) then dried (MgSO₄). The solvent was removed and the residue dissolved in toluene (3 mL) and heated under gentle reflux for 14 h. The cooled mixture was filtered through celite, the filtrate evaporated to dryness and the reisdue purified by flash chromatography (IH-EtOAc 3:2) to give the title compound: δ_{H} (CDCl₃) 1.46 (9H, s), 1.61 (2H, m), 1.69 (3H, d), 1.79 (1H, m), 1.90 (1H, m), 3.11 (3H, s), 3.13 (2H, m), 3.67 (1H, tt), 3.77 (2H, m), 4.96 (1H, q), 8.09 (2H, d), 8.32 (2H, d); RT = 3.76 min, *m*/*z* (ES⁺)= 452.1 [*M*+H]⁺.

### Example 41: 4-{1-[3-(3-Fluoro-4-methylsulfanylphenyl)-[1,2,4]oxadiazol-5-yl]ethoxy}piperidine-1-carboxylic acid tert-butyl ester

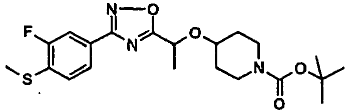

3-Fluoro-*N*-hydroxy-4-methylsulfanylbenzamidine was reacted with 4-(1-carboxyethoxy)piperidine-1-carboxylic acid *tert*-butyl ester using the same procedure as that described in **Example 40** to give the title compound: δ_{H} (CDCl₃) 1.46 (9H, s), 1.60 (2H, m), 1.67 (3H, d), 1.78 (1H, m), 1.90 (1H, m), 2.54 (3H, s), 3.12 (2H, m), 3.65 (1H, tt), 3.77 (2H, m), 4.92 (1H, q), 7.32 (1H, t), 7.76 (1H, dd), 7.86 (1H, dd); RT = 4.27 min, *m*/*z* (ES⁺) = 438.1 [*M*+H]⁺.

### Example 42: 4-{1-[3-(3-Fluoro-4-methoxycarbonylphenyl)-[1,2,4]oxadiazol-5-yl]ethoxy}piperidine-1-carboxylic acid tert-butyl ester

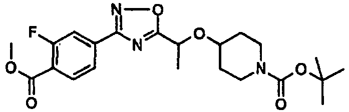

2-Fluoro-4-(*N*-hydroxycarbamimidoyl)benzoic acid methyl ester was reacted with 4-(1-carboxyethoxy)piperidine-1-carboxylic acid *tert*-butyl ester using the same procedure as that described in **Example 40** to give the title compound: δ_{H} (CDCl₃) 1.46 (9H, s), 1.60 (2H, m), 1.68 (3H, d), 1.79 (1H, m), 1.90 (1H, m), 3.12 (2H, m), 3.66 (1H, tt), 3.77 (2H, m), 3.98 (3H, s), 4.95 (1H, q), 7.90 (1H, dd), 7.95 (1H, dd), 8.07 (1H, t); RT = 4.17 min, *m*/*z* (ES⁻) = 450.0 **[*M*+H]⁺.**

### Example 43: 4-{1-[3-(4-Carbamoyl-3-fluorophenyl)-[1,2,4]oxadiazol-5-yl]ethoxy}piperidine-1-carboxylic acid tert-butyl ester

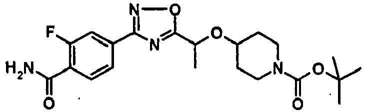

A solution of 4- {1-[3-(3-fluoro-4-methoxycarbonylphenyl)-[1,2,4]oxadiazol-5-yl]ethoxy}piperidine-1-carboxylic acid *tert*-butyl ester (**Example 42**, 430 mg, 958 µmol) in MeOH (3 mL) was treated with 1M aqueous NaOH (2 mL, 2 mmol). After stirring for 3 h, the mixture was acidified with citric acid and diluted with CH₂Cl₂ (20 mL). The organic component was separated and dried (MgSO₄) and evaporated to give 4-{1-[3-(4-carboxy-3-fluorophenyl)-[1,2,4]oxadiazol-5-yl]ethoxy}piperidine-1-carboxylic acid *tert*-butyl ester: RT = 3.69 min, *m*/*z* (ES⁺) = 436.1 [*M*+H]⁺. A sample of this acid (137 mg, 315 µmol), EDC and HOBt were dissolved in anhydrous THF (4 mL) and stirred for 10 min. A solution of ammonia (1.89 mL of a 0.5M solution in dioxane, 945 µmol) was introduced, the reaction mixture stirred at room temperature for 4 h then partitioned between 1M aqueous NaOH (5 mL) and CH₂Cl₂ (20 mL). the organic phase was separated, washed with 1M aqueous HCl (5 mL), water (5 mL) and dried (MgSO₄). The solvent was removed and the residue recrystallised (EtOAc-1H) to afford the title compound: δ_{H} (CDCl₃) 1.46 (9H, s), 1.61 (2H, m), 1.68 (3H, d), 1.79 (1H, m), 1.90 (1H, m), 3.13 (2H, m), 3.67 (1H, tt), 3.77 (2H, m), 4.95 (1H, q), 5.84 (1H, br s), 6.71 (1H, br d), 7.91 (1H, d), 8.03 (1H, d), 8.27 (1H, t); RT = 3.29 min, *m*/*z* (ES⁻) = 435.0 [*M*+H]⁻.

### Example 44: 4-{1-[3-(4-Ethylcarbamoyl-3-fluorophenyl)-[1,2,4]oxadiazol-5-yl]ethoxy}piperidine-1-carboxylic acid tert-butyl ester

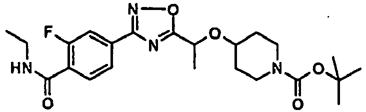

4-{1-[3-(3-Fluoro-4-methoxycarbonylphenyl)-[1,2,4]oxadiazol-5-yl]ethoxy} piperidine-1-carboxylic acid *tert*-butyl ester was saponified and reacted with ethylamine using the procedure described in **Example 43** to afford the title compound: δ_{H} (CDCl₃) 1.30 (3H, t), 1.46 (9H, s), 1.60 (2H, m), 1.68 (3H, d), 1.79 (1H, m), 1.90 (1H, m), 3.13 (2H, m), 3.56 (2H, pentet), 3.66 (1H, tt), 3.76 (2H, m), 4.95 (1H, q), 6.74 (1H, br t), 7.87 (1H, d), 8.00 (1H, d), 8.24 (1H, t); RT = 3.42 min, *m*/*z* (ES⁺) = 463.0 [*M*+H]⁺.

### Examples 45 and 46: 4-{1-[3-(3-Fluoro-4-methanesulfonylphenyl)-[1,2,4]oxadiazol-5-yl]ethoxy}piperidine-1-carboxylic acid tert-butyl ester and 4-{1-[3-(3-fluoro-4-methanesulfinylphenyl)-[1,2,4]oxadiazol-5-yl]ethoxy}piperidine-1-carboxylic acid tert-butyl ester

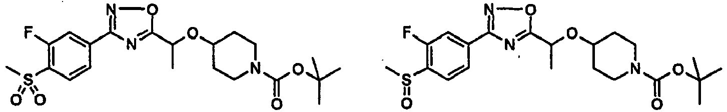

Solid *m*CPBA (140 mg, of 77% purity, 63 µmol) was added in one portion to a stirred solution of 4-{1-[3-(3-fluoro-4-methylsulfanylphenyl)-[1,2,4]oxadiazol-5-yl]ethoxy}piperidine-1-carboxylic acid *tert*-butyl ester (**Example 41**, 183 mg, 420 µmol) in CH₂Cl₂ (3 mL). After 2 h saturated aqueous Na₂CO₃ (15 mL) was added and the mixture extracted with CH₂Cl₂ (3 × 20 mL). The combined extracts were dried (MgSO₄), evaporated and the residue purified by flash chromatography (IH-EtOAc 3:2) to afford firstly the sulfone: δ_{H} (CDCl₃) 1.46 (9H, s), 1.60 (2H, m), 1.68 (3H, d), 1.79 (1H, m), 1.90 (1H, m), 3.13 (2H, m), 3.28 (3H, s), 3.67 (1H, tt), 3.77 (2H, m), 4.95 (1H, q), 8.01 (1H, d), 8.06-8.16 (2H, m); RT = 3.90 min, *m*/*z* (ES⁺) = 470.0 [M+H]⁻; then the sulfoxide: δ_{H} (CDCl₃) 1.46 (9H, s), 1.60 (2H, m), 1.68 (3H, d), 1.79 (1 H, m), 1.90 (1H, m), 2.89 (3H, s), 3.13 (2H, m), 3.67 (1H, tt), 3.77 (2H, m), 4.95 (1H, q), 7.88 (1H, dd), 8.01 (1H, t), 8.15 (1H, dd); RT = 3.67 min, *m*/*z* (ES⁻) = 454.0 [M+H]⁺.

The biological activity of the compounds of the invention may be tested in the following assay systems:

### Yeast Reporter Assay

The yeast cell-based reporter assays have previously been described in the literature (e.g. see Miret J. J. et al, 2002, J. Biol. Chem., 277:6881-6887; Campbell R.M. et al, 1999, Bioorg. Med. Chem. Lett., 9:2413-2418; King K. et al, 1990, Science, 250:121-123; WO 99/14344; WO 00/12704; and US 6,100,042). Briefly, yeast cells have been engineered such that the endogenous yeast G-alpha (GPA1) has been deleted and replaced with G-protein chimeras constructed using multiple techniques. Additionally, the endogenous yeast alpha-cell GPCR, Ste3 has been deleted to allow for a homologous expression of a mammalian GPCR of choice. In the yeast, elements of the pheromone signaling transduction pathway, which are conserved in eukaryotic cells (for example, the mitogen-activated protein kinase pathway), drive the expression of Fus1. By placing β-galactosidase (LacZ) under the control of the Fus1 promoter (Fus1p), a system has been developed whereby receptor activation leads to an enzymatic read-out.

Yeast cells were transformed by an adaptation of the lithium acetate method described by Agatep et al, (Agatep, R. et al, 1998, Transformation of Saccharomyces cerevisiae by the lithium acetate/single-stranded carrier DNA/polyethylene glycol (LiAc/ss-DNA/PEG) protocol. Technical Tips Online, Trends Journals, Elsevier). Briefly, yeast cells were grown overnight on yeast tryptone plates (YT). Carrier single-stranded DNA (10 µg), 2 µg of each of two Fuslp-LacZ reporter plasmids (one with URA selection marker and one with TRP), 2 µg of GPR 119 (human or mouse receptor) in yeast expression vector (2 µg origin of replication) and a lithium acetate/ polyethylene glycol/ TE buffer was pipetted into an Eppendorf tube. The yeast expression plasmid containing the receptor/ no receptor control has a LEU marker. Yeast cells were inoculated into this mixture and the reaction proceeds at 30°C for 60 min. The yeast cells were then heat-shocked at 42°C for 15 min. The cells were then washed and spread on selection plates. The selection plates are synthetic defined yeast media minus LEU, URA and TRP (SD-LUT). After incubating at 30°C for 2-3 days, colonies that grow on the selection plates were then tested in the LacZ assay.

In order to perform fluorimetric enzyme assays for β-galactosidase, yeast cells carrying the human or mouse GPR119 receptor were grown overnight in liquid SD-LUT medium to an unsaturated concentration (i.e. the cells were still dividing and had not yet reached stationary phase). They were diluted in fresh medium to an optimal assay concentration and 90 µL of yeast cells are added to 96-well black polystyrene plates (Costar). Compounds, dissolved in DMSO and diluted in a 10% DMSO solution to 10X concentration, were added to the plates and the plates placed at 30°C for 4 h. After 4 h, the substrate for the β-galactosidase was added to each well. In these experiments, Fluorescein di (β-D-galactopyranoside) was used (FDG), a substrate for the enzyme that releases fluorescein, allowing a fluorimetric read-out. 20 µL per well of 500 µM FDG/2.5% Triton X100 was added (the detergent was necessary to render the cells permeable). After incubation of the cells with the substrate for 60 min, 20 µL per well of 1M sodium carbonate was added to terminate the reaction and enhance the fluorescent signal. The plates were then read in a fluorimeter at 485/535nm.

The compounds of the invention give an increase in fluorescent signal of at lest ~ 1.5-fold that of the background signal (i.e. the signal obtained in the presence of 1% DMSO without compound). Compounds of the invention which give an increase of at least 5-fold may be preferred.

### cAMP Assay

A stable cell line expressing recombinant human GPR119 was established and this cell line was used to investigate the effect of compounds of the invention on intracellular levels of cyclic AMP (cAMP). The cell monolayers were washed with phosphate buffered saline and stimulated at 37°C for 30min with various concentrations of compound in stimulation buffer plus 1% DMSO. Cells were then lysed and cAMP content determined using the Perkin Elmer AlphaScreen™ (Amplified Luminescent Proximity Homogeneous Assay) cAMP kit. Buffers and assay conditions were as described in the manufacturer's protocol.

Compounds of the invention produced a concentration-dependent increase in intracellular cAMP level and generally had an EC₅₀ of <10 µM. Compounds showing an EC₅₀ of less than 1 µM in the cAMP assay may be preferred.

### In vivo feeding study

The effect of compounds of the invention on body weight and food and water intake was examined in freely-feeding male Sprague-Dawley rats maintained on reverse-phase lighting. Test compounds and reference compounds were dosed by appropriate routes of administration (e.g. intraperitoneally or orally) and measurements made over the following 24 h. Rats were individually housed in polypropylene cages with metal grid floors at a temperature of 21±4°C and 55±20% humidity. Polypropylene trays with cage pads were placed beneath each cage to detect any food spillage. Animals were maintained on a reverse phase light-dark cycle (lights off for 8 h from 09.30-17.30 h) during which time the room was illuminated by red light. Animals had free access to a standard powdered rat diet and tap water during a two week acclimatization period. The diet was contained in glass feeding jars with aluminum lids. Each lid had a 3-4 cm hole in it to allow access to the food. Animals, feeding jars and water bottles were weighed (to the nearest 0.1 g) at the onset of the dark period. The feeding jars and water bottles were subsequently measured 1, 2, 4, 6 and 24 h after animals were dosed with a compound of the invention and any significant differences between the treatment groups at baseline compared to vehicle-treated controls.

Selected compounds of the invention showed a statistically significant hypophagic effect at one or more time points at a dose of ≤ 100 mg kg⁻¹.

### Anti-diabetic effects of compounds of the invention in an in-vitro model of pancreatic beta cells (HIT-T15)

### Cell Culture

HIT-T15 cells (passage 60) were obtained from ATCC, and were cultured in RPMI1640 medium supplemented with 10% fetal calf serum and 30 nM sodium selenite. All experiments were done with cells at less than passage 70, in accordance with the literature, which describes altered properties of this cell line at passage numbers above 81 (Zhang HJ, Walseth TF, Robertson RP. Insulin secretion and cAMP metabolism in HIT cells. Reciprocal and serial passage-dependent relationships. Diabetes. 1989 Jan;38(1):44-8).

### cAMP assay

HIT-T15 cells were plated in standard culture medium in 96-well plates at 100,000 cells/ 0.1 mL/ well and cultured for 24 h and the medium was then discarded. Cells were incubated for 15 min at room temperature with 100 µL stimulation buffer (Hanks buffered salt solution, 5 mM HEPES, 0.5 mM IBMX, 0.1% BSA, pH 7.4). This was discarded and replaced with compound dilutions over the range 0.001, 0.003, 0.01, 0.03, 0.1, 0.3, 1, 3, 10, 30 µM in stimulation buffer in the presence of 0.5% DMSO. Cells were incubated at room temperature for 30 min. Then 75 µL lysis buffer (5 mM HEPES, 0.3% Tween-20, 0.1% BSA, pH 7.4) was added per well and the plate was shaken at 900 rpm for 20 min. Particulate matter was removed by centrifugation at 3000rpm for 5min, then the samples were transferred in duplicate to 384-well plates, and processed following the Perkin Elmer AlphaScreen cAMP assay kit instructions. Briefly 25 µL reactions were set up containing 8 µL sample, 5 µL acceptor bead mix and 12 µL detection mix, such that the concentration of the final reaction components is the same as stated in the kit instructions. Reactions were incubated at room temperature for 150 min, and the plate was read using a Packard Fusion instrument. Measurements for cAMP were compared to a standard curve of known cAMP amounts (0.01, 0.03, 0.1, 0.3, 1, 3, 10, 30, 100, 300, 1000 nM) to convert the readings to absolute cAMP amounts. Data was analysed using XLfit 3 software.

Representative compounds of the invention were found to increase cAMP at an EC₅₀ of less than 10 µM. Compounds showing an EC₅₀ of less than 1 µM in the cAMP assay may be preferred.

### Insulin secretion assay

HIT-T15 cells were plated in standard culture medium in 12-well plates at 106 cells/ 1 ml/ well and cultured for 3 days and the medium was then discarded. Cells were washed x 2 with supplemented Krebs-Ringer buffer (KRB) containing 119 mM NaCl, 4.74 mM KCl, 2.54 mM CaCl₂, 1.19 mM MgSO₄, 1.19 mM KH₂PO₄, 25 mM NaHCO₃, 10 mM HEPES at pH 7.4 and 0.1% bovine serum albumin. Cells were incubated with 1ml KRB at 37°C for 30 min which was then discarded. This was followed by a second incubation with KRB for 30 min, which was collected and used to measure basal insulin secretion levels for each well. Compound dilutions (0, 0.1,0.3, 1, 3, 10 µM) were then added to duplicate wells in 1ml KRB, supplemented with 5.6 mM glucose. After 30 min incubation at 37°C samples were removed for determination of insulin levels. Measurement of insulin was done using the Mercodia Rat insulin ELISA kit, following the manufacturers instructions, with a standard curve of known insulin concentrations. For each well insulin levels were corrected by subtraction of the basal secretion level from the pre-incubation in the absence of glucose. Data was analysed using XLfit 3 software.

Representative compounds of the invention were found to increase insulin secretion at an EC₅₀ of less than 10 µM. Compounds showing an EC₅₀ of less than 1µM in the insulin secretion assay may be preferred.

### Oral Glucose Tolerance Tests

The effects of compounds of the invention on oral glucose (Glc) tolerance were evaluated in male C57B1/6 or male *ob*/*ob* mice. Food was withdrawn 5 h before administration of Glc and remained withdrawn throughout the study. Mice had free access to water during the study. A cut was made to the animals' tails, then blood (20 µL) was removed for measurement of basal Glc levels 45 min before administration of the Glc load. Then, the mice were weighed and dosed orally with test compound or vehicle (20% aqueous hydroxypropyl-β-cyclodextrin or 25% aqueous Gelucire 44/14) 30 min before the removal of an additional blood sample (20 µL) and treatment with the Glc load (2-5 g kg⁻¹ p.o.). Blood samples (20 µL) were then taken 25, 50, 80, 120, and 180 min after Glc administration. The 20 µL blood samples for measurement of Glc levels were taken from the cut tip of the tail into disposable micro-pipettes (Dade Diagnostics Inc., Puerto Rico) and the sample added to 480 µL of haemolysis reagent. Duplicate 20 µL aliquots of the diluted haemolysed blood were then added to 180 µL of Trinders glucose reagent (Sigma enzymatic (Trinder) colorimetric method) in a 96-well assay plate. After mixing, the samples were left at room temperature for 30 min before being read against Glc standards (Sigma glucose/urea nitrogen combined standard set). Representative compounds of the invention statistically reduced the Glc excursion at doses ≤ 100 mg kg⁻¹.

## Claims

1. A compound of formula (I), or a pharmaceutically acceptable salt thereof: 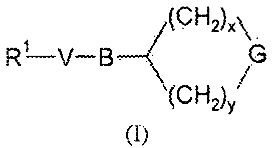 wherein V is a 5-membered heteroaryl ring of the formula: 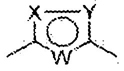 wherein W is N, and one of X and Y is N and the other is O;
B is (CH₂)ₚ-CH(C₁₋₃ alkyl)-(CH₂)_{q}, where one of the CH₂ groups may be replaced by O, NR⁵, S(O)ₘ, C(O), C(O)NR⁵ or CH(NR⁵R⁵⁵);
m is independently 0, 1 or 2;
p + q equals 0, 1 or 2;
x is 2;
y is 2;
G is NR²;
R' is phenyl or a 6-membered heteroaryl group containing up to two N atoms, any of which may be optionally substituted by one or more substituents selected from halo, C₁₋₄alkyl, C₁₋₄ fluoroalkyl, C₁₋₄ hydroxyalkyl, C₃₋₇cycloalkyl, OR⁶, CN, S(O)ₘR⁶, C(O)NR⁶R⁶⁶, NR⁶R⁶⁶, NR¹⁰C(O)R⁶, NR¹⁰SO₂R⁶, SO₂NR⁶R⁶⁶, COR¹⁰, C(O)OR¹⁰, a 4- to 7-membered heterocyclyl group or a 5- or 6-membered, heteroaryl group;
R² is C(O)OR³, C(O)NR³R¹³, S(O)₂R³ or C(O)R³; or monocyclic 5- or 6- membered heteroaryl ring containing upto 4 heteroatoms selected from N, O and S, which may optionally be substituted by one or two groups selected from C₁₋₄alkyl, C₁₋₄alkoxy and halogen;
R³ is C₁₋₈alkyl, C₂₋₈alkenyl or C₂₋₈alkynyl, any of which may be optionally substituted by one or more halo atoms, NR⁴R⁴⁴, OR⁴, C(C)OR⁴, OC(O)R⁴ or CN groups, and may contain a CH₂ group that is replaced by O or S; or C₃₋₇ cycloalkyl, aryl, 4 to 7 memembered heterocyclyl ring containing up to 3 heteroatoms selected from N, O and S, 6 membered heteroaryl ring containing up to 4 heteroatoms selected from N, O or S, C₁₋₄akylene, C₃₋₇ cycloalkyl, C₁₋₄ alkylenearyl, C₁₋₄ alkyleneheterocyclyl or C₁₋₄ alkyleneheteroaryl, any of which may be substituted with one or more substituents selected from halo, C₁₋₄alkyl, C₁₋₄ fluoroalkyl, OR⁴, CN, NR⁴R⁴⁴_{,} SO₂Me, NO₂ or C(O)OR⁴;
R⁴ and R⁴⁴ are independently hydrogen or C₁₋₄alkyl; or, taken together, R⁴ and R⁴⁴ may form a 5- or 6-membered heterocyclic ring;
R⁵ and R⁵⁵ independently represent hydrogen or C₁₋₄alkyl;
R⁶ and R⁶⁶ are independently hydrogen or C₁₋₄ alkyl, which may optionally be substituted by halo, hydroxy, C₁₋₄alkyloxy-, C₁₋₄alkylthio-, C₃₋₇heterocyclyl or N(R¹⁰)₂; or C₃₋₇ cycloalkyl, aryl, heterocyclyl or heteroaryl, wherein the cyclic groups may be substituted with one or more substituents selected from halo, C₁₋₄ alkyl, C₁₋₄ fluoroalkyl, OR⁹, CN, SO₂CH₃, N(R¹⁰)₂ and NO₂; or, taken together, R⁶ and R⁶⁶ may form a 5- or 6-membered heterocyclic ring optionally substituted by hydroxy, C₁₋₄ alkyl or C₁₋₄ hydroxyalkyl; or R⁶⁶ is C₁₋₄ alkyloxy-;
R⁹ is hydrogen, C₁₋₂ alkyl or C₁₋₂ fluoroalkyl;
R¹⁰ are independently hydrogen or C₁₋₄ alkyl; or a group N(R¹⁰)₂ may form a 4- to 7-membered heterocyclic ring optionally containing a further heteroatom selected from O and NR¹⁰; and
R¹³ is hydrogen or C₁₋₄alkyl.

2. A compound according to any one of the preceding claims, or a pharmaceutically acceptable salt thereof, wherein in B, p + q is 1 or 2.

3. A compound according to claim 2, or a pharmaceutically acceptable salt thereof, wherein in B, p + q is 1.

4. A compound according to any one of the preceding claims, or a pharmaceutically acceptable salt thereof, wherein B is -CH(CH₃)-O-.

5. A compound according to claim 4, or a pharmaceutically acceptable salt thereof, wherein the absolute configuration at the chiral carbon atom in -CH(CH₃)-O- is (R).

6. A compound according to any one of the preceding claims, or a pharmaceutically acceptable salt thereof, wherein R¹ is phenyl.

7. A compound according to any one of the preceding claims, or a pharmaceutically acceptable salt thereof, wherein R² is C(O)OR³, CCO)NR³R¹³ or monocyclic 5- or 6- membered heleroaryl ring containing up to 4 heteroatoms selected from N, O and S.

8. A compound according to claim 7, or a pharmaceutically acceptable salt thereof, wherein R² is C(O)OR³.

9. A compound according to any one of the preceding claims, or a pharmaceutically acceptable salt thereof, wherein R³ represents C₃₋₅alkyl, optionally substituted by one or more halo atoms or CN, and may contain a CH₂ group that is replaced by O or S, or C₃₋₅cycloalkyl, optionally substituted by halo, C₁₋₄ alkyl, C₁₋₄ fluoroalkyl,. OR⁴, CN, NR⁴R⁴⁴, NO₂ or CCO)OC₁. ₄alkyl.

10. A compound of formula (I) selected from:
4-{2-[3-(3-Fluoro-4-methylsulfanylphenyl)-[1,2,4]joxadiaxol-5-yl]-1-methylethyl}piperidine-1-carboxylic acid *tert*-butyl ester;
4-{2-[3-(3-Fluoro-4-methylsulfanylphenyl)-[1,2,4]oxadiazol-5-yl]propyl}piêridine-1-carboxylic acid *tert*-butyl ester;
4-{1-[3-(3-Fluoro-4-methylsulfanylphenyl)-[1,2,4]oxadiazol-5-yl]propoxy}piperidine-1-carboxylic acid *tert*-butyl ester;
4-{(*S*)-1-[3-(3-Fluoro-4-methylsulfanylphenyl)-[1,2,4]oxadiazol-5-yl]ethoxy}piperidine-1-carboxylic acid *tert*-butyl ester;
4-{(*R*)-1-[3-(3-Fluoro-4-methylsulfanylphenyl)-[1,2,4]oxadiazol-5-yl]ethoxy}piperidine-1-carboxylic acid *tert*-butyl ester;
4-{1-[3-(3,5-Difluoro-4-methylsulfanylphenyl)-[1,2,4]oxadiaxol-5-yl]-ethoxy}piperidine-1-carboxylic acid *tert*-butyl ester;
1-(3-Isopropyl-[1,2,4]oxadiazol-5-yl)-4-{(*R*)-1-[3-(4-methylsulfanylphenyl)-[1,2,4]oxadiazol-5-yl]ethoxy}piperidine;
4-{(*R*)-1-[3-(3-Fluoro-4-methylsulfanylphenyl)-[1,2,4]oxadiazol-5-yl]ethoxy}-1-(3-isopropyl-[1,2,4]oxadiazol-5-yl)piperidine;
1-(3-Isopropyl-[1,2,4]oxadiazol-5-yl)-4-{(*R*)-1-[3-(3-methysulfanylphenyl)-[1,2,4]oxadiazol-5-yl]ethoxy} piperidine;
1-(5-Isopropyl-[1,2,4]oxadiazol-3-yl)-4-{(*R*)-1-[3-(4-methylsulfanylphenyl)-[1,2,4]oxadiazol-5-yl]ethoxy}piperidine;
4-{(*R*)-1-[3-(3-Fluoro-4-methylfulfanylphenyl)-[1,2,4]oxadiazol-5-yl]ethoxy}-1-(5-isopropyl-[1,2,4]oxadiazol-3-yl)piperidine;
1-(5-Isopropyl-[1,2,4]oxadiazol-3-yl)-4-{(*R*)-1-[3-(3-methyl-4-methylsulfanylphenyl)-[1,2,4]oxadiazol-5-yl]ethoxy}piperidine;
4-(5-{(*R*)-1-[1-(3-Isopropyl-[1,2,4]oxadiazol-5-yl)piperidin-4-yloxy]ethyl}-[1,2,4]oxadiazol-3-yl)pyridine;
4-(5-{(*R*)-1-[1-(3-Isopropyl-[1,2,4]oxadiazol-5-yl)piperidin-4-yloxy]ethyl}-[1,2,4]oxadiazol-3-yl)-2-methylpyridine;
1-[(*R*)-1-(3-Pyridin-4-yl-[1,2,4]oxadiazol-5-yl)ethoxy]piperidine-1-carboxylic acid *tert-*butyl ester;
4-{(*R*)-1-[3-(2-Methylpyridin-4-yl)-[1,2,4]oxadiazol-5-yl]ethoxy}piperidine-1-carboxylic acid *tert*-butyl ester;
1-(-*tert*-Butyl-[1,2,4]oxadiazol-5-yl)-4-{(*R*)-1-[3-(-fluoro-4-methylsulfanylphenyl)-[1,2,4]oxadiazol-5-yl]ethoxy} piperidine;
4-{1-[3-(3-Fluoro-4-methanesulfinylphenyl)-[1,2,4]oxadiazol-5-yl]propoxy} piperidine-1-carboxylic acid *tert*-butyl ester;
4-{2-[3-(3-Fluoro-4-methanesulfinylphenyl)-[1,2,4]oxadiazol-5-yl]-1-methylethyl}-piperidine-1-carboxylic acid *tert*-butyl ester;
4-{2-[3-(3-Fluoro-4-methanesulfonylphenyl)-[1,2,4]oxadiazol-5-yl]-1-methylethyl}-piperidine-1-carboxylic acid *tert*-butyl ester;
4-{2-[3-(3-Fluoro-4-methanesulfinylphenyl)-[1,2,4]oxadiazol-5-yl]propyl}piperidine-1-carboxylic acid *tert*-butyl ester;
4-{2-[3-(3-Fluoro-4-methanesulfonylphenyl)-[1,2,4]oxadiazol-5-yl]propyl}piperidine-1-carboxylic acid *tert*-butyl ester;
4-{(*S*)-1-[3-(3-Fluoro-4-methanesulfonylphenyl)-[1,2,4]oxadiazol-5-yl]ethoxy}piperidine-1-carboxylic acid *tert*-butyl ester;
4-{(*R*)-1-[3-(3-Fluoro-4-methanesulfonylphenyl)-[1,2,4]oxadiazol-5-yl] ethoxy}piperidine-1-carboxylic acid *tert*-butyl ester;
4-{(-[3-(3-Fluoro-4-methanesulfinylphenyl)-[1,2,4]oxadiazol-5-yl] propoxy}piperidine-1-carboxylic acid *tert*-butyl ester;
4-{1-[3-(3-Fluoro-4-methanesulfonylphenyl)-[1,2,4]oxadiazol-5-yl] propoxy}piperidine-1-carboxylic acid *tert*-butyl ester;
4-{1-[3-(3,5-Difluoro-4-methanesulfinylphenyl)-[1,2,4]oxadiazol-5-yl]ethoxy} piperidine-1-carboxylic acid *tert*-butyl ester;
4-{1-[3-(3,5-Difluoro-4-methanesulfonylphenyl)-[1,2,4]oxadiazol-5-yl]ethoxy} piperidine-1-carboxylic acid *tert*-butyl ester;
1-(3-*tert*-Butyl-[1,2,4]oxadiazol-5-yl)-4-{(*R*)-1-[3-(3-fluoro-4-methanesulfinylphenyl)-[1,2,4]oxadiazol-5-yl]ethoxy}piperidine;
1-(3-*tert*-Butyl-[1,2,4]oxadiazol-5-yl)-4-{(*R*)-1-[3-(3-fluoro-4-methansulfonylphenyl)-[1,2,4]oxadiazol-5-yl]ethoxy}piperidine;
1-(3-Isopropyl-[1,2,4]oxadiazol-5-yl)-4-{(*R*)-1-[3-(4-methanesulfonylphenyl)-[1,2,4] oxadiazol-5-yl]ethoxy}piperidine;
4-{(*R*)-1-[3-(3-Fluoro-4-methanesulfonylphenyl)-[1,2,4]oxadiazol-5-yl]ethoxy}-1-(3-isopropyl-[1,2,4]oxadiazol-5-yl)piperidine;
1-(3-Isopropyl-[1,2,4]oxadiazol-5-yl)-4-{(*R*)-1-[3-(4-methylphenyl)-[1,2,4]oxadiazol-5-yl]ethoxy}piperidine;
1-(5-Isopropyl-[1,2,4]oxadiazol-3-yl)-4-{(*R*)-1-[3-(4-methanesulfonylphenyl)-[1,2,4]oxadiazol-5-yl]ethoxy}piperidine;
4-{(*R*)-1-[3-(3-Fluoro-4-methanesulfonylphenyl)-[1,2,4]oxadiazol-5-yl]ethoxy}-1-(5-isopropyl-[1,2,4]oxadiazol-3-yl)piperidine;
1-(5-Isopropyl-[1,2,4]oxadiazol-3-yl)-4-{(*R*)-1-[3-(4-methanesulfonyl-3-methylphenyl)-[1,2,4]oxadiazol-5-yl]ethoxy}piperidine;
4-{1-[3-(3-Fluoro-4-sulfamoylphenyl)-[1,2,4]oxadiaxol-5-yl]ethoxy}piperidine-1-carboxylic acid *tert*-butyl ester;
4-{(*R*)-1-[3-(3-Fluoro-4-sulfamoylphenyl)[1,2,4]oxadiazol-5-yl]ethoxy}piperidine-1-carboxylic acid *tert*-butyl ester;
4-{(*R*)-1-[3-(3-Fluoro-4-methanesulfonylphenyl)-[1,2,4]oxadiazol-5-yl]ethoxy}piperidine-1-carboxylic acid isopropyl ester;
4-{1-[3-(4-Methanesulfonylphenyl)-[1,2,4]oxadiazol-5-yl]ethoxy}piperidine-1-carboxylic acid *tert*-butyl ester;
4-{1-[3-(3-Fluoro-4-methylsulfanylphenyl)-[1,2,4]oxadiazol-5-yl]ethoxy}piperidine-1-carboxylic acid *tert*-butyl ester;
4-{1-[3-(3-Fluoro-4-methoxycarbonylphenyl)-[1,2,4]oxadiaxol-5-yl]ethoxy}piperidine-1-carboxylic acid *tert*-butyl ester;
4-{1-[3-(4-Carbamoyl-3-fluorophenyl)-[1,2,4]oxadiazol-5-yl]ethoxy}piperidine-1-carboxylic acid *tert*-butyl ester;
4-{1-[3-(4-Ethylcarbamoyl-3-fluorophenyl)-[1,2,4]oxadiaxol-5-yl]ethoxy}piperidine-1-carboxylic acid *tert*-butyl ester;
4-{1-[3-(3-Fluoro-4-methanesulfonylphenyl)-[1,2,4]oxadiazol-5-yl]ethoxy}piperidine-1-carboxylic acid *tert*-butyl ester; and
4-{1-[3-(3-Fluoro-4-methansulfinylphenyl)-[1,2,4]oxadiazol-5-yl]ethoxy}piperidine-1-carboxylic acid *tert*-butyl ester;
or a pharmaceutically acceptable salt of any one thereof.

11. A pharmaceutical composition comprising a compound according to any one of claims 1 to 10, or a pharmaceutically acceptable salt thereof, and a pharmaceutically acceptable carrier.

12. A compound according to any one of claims 1 to 10, or a pharmaceutically acceptable salt thereof, for use in the treatment of diabetes; obesity; metabolic syndrome; type 1 and type 2 diabetes; impaired glucose tolerance; insulin resistance; diabetic complications such as neuropathy, nephropathy, retinopathy, cataracts, cardiovascular complications, dyslipidaemia; abnormal sensitivity to ingested fats leading to functional dyspepsia; and metabolic diseases such as metabolic syndrome, impaired glucose tolerance, hyperlipidemia, hypertriglyceridemia, hypercholesterolemia low HDL, levels and hypertension.

13. A compound according to any one of claims 1 to 10, or a pharmaceutically acceptable salt thereof, for use in the regulation of satiety or the treatment of obesity.

14. A compound according to any one of claims 1 to 10, or a pharmaceutically acceptable salt thereof, for use in the treatment of diabetes.

15. A compound according to any one of claims 1 to 10, or a pharmaceutically acceptable salt thereof, for use in the treatment of metabolic syndrome (syndrome X), impaired glucose tolerance, hyperlipidemia, hypertriglyceridemia, hypercholesterolemia, low HDL levels or hypertension.

16. A compound of formula (XXVII): 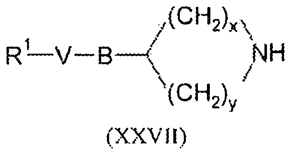 wherein V is a 5-membered heteroaryl ring of the formula: 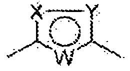 wherein W is N, and one of X and Y is N and the other is O;
B is (CH₂)ₚ-CH(C₁₋₃ alkyl)-(CH₂)_{q}, where one of the CH₂ groups may be replaced by O, NR⁵, S(O)ₘ, C(O), C(O)NR₅, CH(NR⁵R⁵⁵), C(O)O, C(O)S, SC(O) or OC(O);
m is independently 0, 1 or 2;
p + q equals 0, 1 or 2;
x is 2;
y is 2;
R¹ is phenyl or a 6-membered heteroaryl group containing up to two N atoms, any of which may be optionally substituted by one or more substituents selected from halo, C₁₋₄ alkyl, C₁₋₄ fluoroalkyl, C₁₋₄ hydroxyalkyl, C₂₋₄ alkenyl, C₂₋₄ alkynyl, C₃₋₇ cycloalkyl, aryl, OR⁶, CN, NO₂, S(O)ₘR⁶, C(O)NR⁶R⁶⁶, NR⁶R⁶⁶, NR¹⁰C(O)R⁶, NR¹⁰SO₂R⁶, SO₂NR⁶R⁶⁶, COR¹⁰, C(O)OR¹⁰, a 4- to 7-membered heterocyclyl group or a 5- or 6-membered heteroaryl group, provided that R¹ is not 4-fluoroalkylpyrid-3-yl or 4-fluoroalkylpyrimidin-5-yl;
R⁵ and R⁵⁵ independently represent hydrogen or C₁₋₄ alkyl;
R⁶ and R⁶⁶ are independently hydrogen or C₁₋₄ alkyl, which may optionally be substituted by halo, hydroxy, C₁₋₄ alkyloxy-, C₁₋₄ alkylthio-, C₃₋₇ heterocyclyl or N(R¹⁰)₂; or C₃₋₇ cycloalkyl, aryl, heterocyclyl or heteroaryl, wherein the cyclic groups may be substituted with one or more substituents selected from halo, C₁₋₄ alkyl, C₁₋₄ fluoroalkyl, OR⁹, CN, SO₂CH₃, N(R¹⁰)₂ and NO₂; or, taken together, R⁶ and R⁶⁶ may from a 5- or 6-membered heterocyclic ring optionally substituted by hydroxy, C₁₋₄ alkyl or C₁₋₄ hydroxyalkyl; or R⁶⁶ is C₁₋₄ alkyloxy-; and
R¹⁰ are independently hydrogen or C₁₋₄ alkyl; or a group N(R¹⁰)₂ may form a 4- to 7-membered heterocyclic ring optionally containing a further heteroatom selected from O and NR¹⁰.

## Patentansprüche

1. Verbindung der Formel (I) oder ein pharmazeutisch verträgliches Salz davon 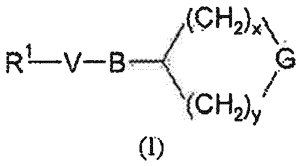 wobei V ein 5-gliedriger Heteroarylring der Formel 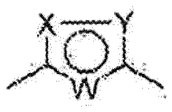 ist,
worin W N ist und einer von X und Y N und der andere O ist,
B (CH₂)ₚ-CH(C₁₋₃-Alkyl)-(CH₂)_{q} ist, worin eine der CH₂-Gruppen durch O, NR⁵, S(O)ₘ, C(O), C(O)NR⁵ oder CH(NR⁵R⁵⁵) ersetzt sein kann,
m unabhängig 0, 1 oder 2 ist,
p + q gleich 0, 1 oder 2 ist,
x 2 ist,
y 2 ist,
G NR² ist,
R¹ Phenyl oder eine 6-gliedrige Heteroarylgruppe ist, die bis zu zwei N-Atome enthält, wobei jede davon gegebenenfalls mit einem oder mehreren Substituenten substituiert sein kann, der oder die aus Halogen, C₁₋₄-Alkyl, C₁₋₄-Fluoralkyl, C₁₋₄-Hydroxyalkyl, C₃₋₇-Cycloalkyl, OR⁶, CN, S(O)ₘR⁶, C(O)NR⁶R⁶⁶, NR⁶R⁶⁶, NR¹⁰C(O)R⁶, NR¹⁰SO₂R⁶, SO₂NR⁶R⁶⁶, COR¹⁰, C(O)OR¹⁰, einer 4- bis 7-gliedrigen Heterocyclylgruppe oder einer 5- oder 6-gliedrigen Heteroarylgruppe ausgewählt ist oder sind,
R² C(O)OR³, C(O)NR³R¹³, S(O)₂R³ oder C(O)R³, oder ein monocyclischer 5- oder 6-gliedriger Heteroarylring ist, der bis zu 4 Heteroatome enthält, die aus N, O und S ausgewählt sind, und der gegebenenfalls durch eine oder zwei Gruppe(n) substituiert sein kann, die aus C₁₋₄-Alkyl, C₁₋₄-Alkoxy und Halogen ausgewählt ist oder sind,
R³ C₁₋₈-Alkyl, C₂₋₈-Alkenyl oder C₂₋₈-Alkinyl ist, wobei jedes davon gegebenenfalls durch ein(e) oder mehrere Halogenatom(e), NR⁴R⁴⁴-, OR⁴-, C(O)OR⁴-, OC(O)R⁴- oder CN-Gruppe(n) substituiert sein kann, und eine CH₂-Gruppe enthalten kann, die durch O oder S ersetzt ist, oder C₃₋₇-Cycloalkyl, Aryl, ein 4- bis 7-gliedriger Heterocyclylring, der bis zu 3 Heteroatome enthält, die aus N, O und S ausgewählt sind, ein 6-gliedriger Heteroarylring, der bis zu 4 Heteroatome enthält, die aus N, O oder S ausgewählt sind, C₁₋₄-Alkylen, C₃₋₇-Cycloalkyl, C₁₋₄-Alkylenaryl, C₁₋₄-Alkylenheterocyclyl oder C₁₋₄-Alkylenheteroaryl ist, wobei jeder davon mit einem oder mehreren Substituenten substituiert sein kann, der oder die aus Halogen, C₁₋₄-Alkyl, C₁₋₄-Fluoralkyl, OR⁴, CN, NR⁴R⁴⁴, SO₂Me, NO₂ oder C(O)OR⁴ ausgewählt ist oder sind,
R⁴ und R⁴⁴ unabhängig Wasserstoff oder C₁₋₄-Alkyl sind oder R⁴ und R⁴⁴ zusammengenommen einen 5- oder 6-gliedrigen heterocyclischen Ring bilden können,
R⁵ und R⁵⁵ unabhängig Wasserstoff oder C₁₋₄-Alkyl darstellen,
R⁶ und R⁶⁶ unabhängig Wasserstoff oder C₁₋₄-Alkyl sind, das gegebenenfalls durch Halogen, Hydroxy, C₁₋₄-Alkyloxy-, C₁₋₄-Alkylthio-, C₃₋₇-Heterocyclyl oder N(R¹⁰)₂ substituiert sein kann, oder C₃₋₇-Cycloalkyl, Aryl, Heterocyclyl oder Heteroaryl sind, wobei die cyclischen Gruppen mit einem oder mehreren Substituenten substituiert sein können, der oder die aus Halogen, C₁₋₄-Alkyl, C₁₋₄-Fluoralkyl, OR⁹, CN, SO₂CH₃, N(R¹⁰)₂ und NO₂ ausgewählt ist oder sind, oder R⁶ und R⁶⁶ zusammengenommen einen 5- oder 6-gliedrigen heterocyclischen Ring bilden können, der gegebenenfalls durch Hydroxy, C₁₋₄-Alkyl oder C₁₋₄-Hydroxyalkyl substituiert ist, oder R⁶⁶ C₁₋₄-Alkyloxy- ist,
R⁹ Wasserstoff, C₁₋₂-Alkyl oder C₁₋₂-Fluoralkyl ist,
R¹⁰ unabhängig Wasserstoff oder C₁₋₄-Alkyl sind oder eine Gruppe N(R¹⁰)₂ einen 4-bis 7-gliedrigen heterocyclischen Ring bilden kann, der gegebenenfalls ein weiteres Heteroatom enthält, das aus O und NR¹⁰ ausgewählt ist, und
R¹³ Wasserstoff oder C₁₋₄-Alkyl ist.

2. Verbindung nach Anspruch 1 oder ein pharmazeutisch verträgliches Salz davon, wobei in B p + q 1 oder 2 ist.

3. Verbindung nach Anspruch 2 oder ein pharmazeutisch verträgliches Salz davon, wobei in B p + q 1 ist.

4. Verbindung nach einem der vorhergehenden Ansprüche oder ein pharmazeutisch verträgliches Salz davon, wobei B -CH(CH₃)-O- ist.

5. Verbindung nach Anspruch 4 oder ein pharmazeutisch verträgliches Salz davon, wobei die absolute Konfiguration am chiralen Kohlenstoffatom in -CH(CH₃)-O- (*R*) ist.

6. Verbindung nach einem der vorhergehenden Ansprüche oder ein pharmazeutisch verträgliches Salz davon, wobei R¹ Phenyl ist.

7. Verbindung nach einem der vorhergehenden Ansprüche oder ein pharmazeutisch verträgliches Salz davon, wobei R² C(O)OR³, C(O)NR³R¹³ oder ein monocyclischer 5- oder 6-gliedriger Heteroarylring ist, der bis zu 4 Heteroatome enthält, die aus N, O und S ausgewählt sind.

8. Verbindung nach Anspruch 7 oder ein pharmazeutisch verträgliches Salz davon, wobei R² C(O)OR³ ist.

9. Verbindung nach einem der vorhergehenden Ansprüche oder ein pharmazeutisch verträgliches Salz davon, wobei R³ C₃₋₅-Alkyl darstellt, das gegebenenfalls durch ein oder mehrere Halogenatom(e) oder CN substituiert ist, und eine CH₂-Gruppe enthalten kann, die durch O oder S ersetzt ist, oder C₃₋₅-Cycloalkyl darstellt, das gegebenenfalls durch Halogen, C₁₋₄-Alkyl, C₁₋₄-Fluoralkyl, OR⁴, CN, NR⁴R⁴⁴, NO₂ oder C(O)OC₁₋₄-Alkyl substituiert ist.

10. Verbindung gemäß Formel (I), ausgewählt aus:
4-{2-[3-(3-Fluor-4-methylsulfanylphenyl)-[1,2,4]oxadiazol-5-yl]-1-methylethyl}piperidin-1-carbonsäure-tert-butylester,
4-{2-[3-(3-Fluor-4-methylsulfanylphenyl)-[1,2,4]oxadiazol-5-yl]propyl}piperidin-1-carbonsäure-tert-butylester,
4-{1-[3-(3-Fluor-4-methylsulfanylphenyl)-[1,2,4]oxadiazol-5-yl]propoxy}piperidin-1-carbonsäure-tert-butylester,
4-{(*S*)-1-[3-(3-Fluor-4-methylsulfanylphenyl)-[1,2,4]oxadiazol-5-yl]ethoxy}piperidin-1-carbonsäure-tert-butylester,
4-{(*R*)-1-[3-(3-Fluor-4-methylsulfanylphenyl)-[1,2,4]oxadiazol-5-yl]ethoxy}piperidin-1-carbonsäure-tert-butylester,
4-{1-[3-(3,5-Difluor-4-methylsulfanylphenyl)-[1,2,4]oxadiazol-5-yl]ethoxy}piperidin-1-carbonsäure-tert-butylester,
1-(3-isopropyl-[1,2,4]oxadiazol-5-yl]-4-{(*R*)-1-[3-(4-methylsulfanylphenyl)-[1,2,4]oxadiazol-5-yl]ethoxy}piperidin,
4-{(*R*)-1-[3-(3-Fluor-4-methylsulfanylphenyl)-[1,2,4]oxadiazol-5-yl]ethoxy}-1-(3-isopropyl-[1,2,4]oxadiazol-5-yl)piperidin,
1-(3-Isopropyl-[1,2,4]oxadiazol-5-yl)-4-{(*R*)-1-[3-(3-methyl-4-methylsulfanylphenyl)-[1,2,4]oxadiazol-5-yl]ethoxy}piperidin,
1-(5-Isopropyl-[1,2,4]oxadiazol-3-yl)-4-{(*R*)-1-[3-(4-methylsulfanylphenyl)-[1,2,4]oxadiazol-5-yl]ethoxy}piperidin,
4-{(*R*)-1-[3-(3-Fluor-4-methylsulfanylphenyl)-[1,2,4]oxadiazol-5-yl]ethoxy}-1-(5-isopropyl-[1,2,4]oxadiazol-3-yl)piperidin,
1-(5-Isopropyl-[1,2,4]oxadiazol-3-yl)-4-{(*R*)-1-[3-(3-methyl-4-methylsulfanylphenyl)-[1,2,4]oxadiazol-5-yl]ethoxy}piperidin,
4-(5-{(*R*)-1-[1-(3-Isopropyl-[1,2,4]oxadiazol-5-yl)piperidin-4-yloxylethyl}-[1,2,4]oxadiazol-3-yl)pyridin,
4-(5-{(*R*)-1-[1-(3-Isopropyl-[1,2,4]oxadiazol-5-yl)piperidin-4-yloxy]ethyl}-[1,2,4]oxadiazol-3-yl)-2-methylpyridin,
4-[(*R*)-1-(3-Pyridin-4-yl-[1,2,4]oxadiazol-5-yl]ethoxy]piperidin-1-carbonsäure-tert-butylester,
4-{(*R*)-1-[3-(2-Methylpyridin-4-yl)-[1,2,4]oxadiazol-5-yl]ethoxy}piperidin-1-carbonsäure-tert-butylester,
1-(3-tert-Butyl-[1,2,4]oxadiazol-5-yl]-4-{(*R*)-1-[3-(3-fluor-4-methylsulfanylphenyl)-[1,2,4]oxadiazol-5-yl]ethoxy}piperidin,
4-{1-[3-(3-Fluor-4-methansulfinylphenyl)-[1,2,4]oxadiazol-5-yl]propoxy}piperidin-1-carbonsäure-tert-butylester,
4-{2-[3-(3-Fluor-4-methansulfinylphenyl)-[1,2,4]oxadiazol-5-yl]-1-methylethyl}-piperidin-1-carbonsäure-tert-butylester,
4-{2-[3-(3-Fluor-4-methansulfonylphenyl)-[1,2,4]oxadiazol-5-yl]-1-methylethyl}-piperidin-1-carbonsäure-tert-butylester,
4-{2-[3-(3-Fluor-4-methansulfinylphenyl)-[1,2,4]oxadiazol-5-yl]propyl}piperidin-1-carbonsäure-tert-butylester,
4-{2-[3-(3-Fluor-4-methansulfonylphenyl)-[1,2,4]oxadiazol-5-yl]propyl}piperidin-1-carbonsäure-tert-butylester,
4-{(*S*)-1-[3-(3-Fluor-4-methansulfonylphenyl)-[1,2,4]oxadiazol-5-yl]ethoxy}piperidin-1-carbonsäure-tert-butylester,
4-{(*R*)-1-[3-(3-Fluor-4-methansulfonylphenyl)-[1,2,4]oxadiazol-5-yl]ethoxy}piperidin-1-carbonsäure-tert-butylester,
4-{1-[3-(3-Fluor-4-methansulfinylphenyl)-[1,2,4]oxadiazol-5-yl]propoxy}piperidin-1-carbonsäure-tert-butylester,
4-{1-[3-(3-Fluor-4-methansulfonylphenyl)-[1,2,4]oxadiazol-5-yl]propoxy}piperidin-1-carbonsäure-tert-butylester,
4-{1-[3-(3,5-Difluor-4-methansulfinylphenyl)-[1,2,4]oxadiazol-5-yl]ethoxy}piperidin-1-carbonsäure-tert-butylester,
4-{1-[3-(3,5-Difluor-4-methansulfonylphenyl)-[1,2,4]oxadiazol-5-yl]ethoxy}piperidin-1-carbonsäure-tert-butylester,
1-(3-tert-Butyl-[1,2,4]oxadiazol-5-yl)-4-{(*R*)-1-[3-(3-fluor-4-methansulfinylphenyl)-[1,2,4]oxadiazol-5-yl]ethoxy}piperidin,
1-(3-tert-Butyl-[1,2,4]oxadiazol-5-yl)-4-{(*R*)-1-[3-(3-fluor-4-methansulfonylphenyl)-[1,2,4]oxadiazol-5-yl]ethoxy}piperidin,
1-(3-Isopropyl-[1,2,4]oxadiazol-5-yl)-4-{(*R*)-1-[3-(4-methansulfonylphenyl)-[1,2,4]oxadiazol-5-yl]ethoxy}piperidin,
4-{(*R*)-1-[3-(3-Fluor-4-methansulfonylphenyl)-[1,2,4]oxadiazol-5-yl]ethox}-1-(3-isopropyl-[1,2,4]oxadiazol-5-yl)piperidin,
1-(3-Isopropyl-[1,2,4]oxadiazol-5-yl)-4-{(*R*)-1-[3-(4-methansulfonyl-3-methylphenyl)-[1,2,4]oxadiazol-5-yl]ethoxylpiperidin,
1-(5-Isopropyl-[1,2,4]oxadiazol-3-yl)-4-{(*R*)-1-[3-(4-methansulfonylphenyl)-[1,2,4]oxadiazol-5-yl]ethoxy}piperidin,
4-{(*R*)-1-[3-(3-Fluor-4-methansulfonylphenyl)-[1,2,4]oxadiazol-5-yl]ethoxy}-1-(5-isopropyl-[1,2,4]oxadiazol-3-yl)piperidin,
1-(5-Isopropyl-[1,2,4]oxadiazol-3-yl)-4-{(*R*)-1-[3-(4-methansulfonyl-3-methylphenyl)-[1,2,4]oxadiazol-5-yl]ethoxy}piperidin,
4-{1-[3-(3-Fluor-4-sulfamoylphenyl)-[1,2,4]oxadiazol-5-yl]ethoxy}piperidin-1-carbonsäure-tert-butylester,
4-{(*R*)-1-[3-(3-Fluor-4-sulfamoylphenyl)-[1,2,4]oxadiazol-5-yl]ethoxy}piperidin-1-carbonsäure-tert-butylester,
4-{(*R*)-1-[3-(3-Fluor-4-methansulfonylphenyl)-[1,2,4]oxadiazol-5-yl]ethoxy}piperidin-1-carbonsäure-isopropylester,
4-{1-[3-(4-Methansulfonylphenyl)-[1,2,4]oxadiazol-5-yl]ethoxy}piperidin-1-carbonsäure-tert-butylester,
4-{1-[3-(3-Fluor-4-methansulfanylphenyl)-[1,2,4]oxadiazol-5-yl]ethoxy}piperidin-1-carbonsäure-tert-butylester,
4-{1-[3-(3-Fluor-4-methoxycarbonylphenyl)-[1,2,4]oxadiazol-5-yl]ethoxy}piperidin-1-carbonsäure-tert-butylester,
4-{1-[3-(4-Carbamoyl-3-fluorphenyl)-[1,2,4]oxadiazol-5-yl]ethoxy}piperidin-1-carbonsäure-tert-butylester,
4-{1-[3-(4-Ethylcarbamoyl-3-fluorphenyl)-[1,2,4]oxadiazol-5-yl]ethoxy}piperldln-1-carbonsäure-tert-butylester,
4-{1-[3-(3-Fluor-4-methansulfonylphenyl)-[1,2,4]oxadiazol-5-yl]ethoxy}piperidin-1-carbonsäure-tert-butylester und
4-{1-[3-(3-Fluor-4-methansulfinylphenyl)-[1,2,4]oxadiazol-5-yl]ethoxy}piperidin-1-carbonsäure-tert-butylester,
oder einem pharmazeutisch verträglichen Salz von einem davon.

11. Pharmazeutische Zusammensetzung, die eine Verbindung nach einem der Ansprüche 1 bis 10 oder ein pharmazeutisch verträgliches Salz davon und einen pharmazeutisch verträglichen Träger umfasst.

12. Verbindung nach einem der Ansprüche 1 bis 10 oder ein pharmazeutisch verträgliches Salz davon zur Verwendung bei der Behandlung von Diabetes, Fettsucht, Stoffwechselsyndrom, Typ 1- und Typ 2-Diabetes, beeinträchtigter Glukosetoleranz, Insulinresistenz, diabetischen Komplikationen, wie z.B. Neuropathie, Nephropathie, Retinopathie, grauer Star, kardiovaskulären Komplikationen, Dyslipidämie, anomaler Sensibilität bezüglich aufgenommenen Fetten, die zu einer funktionellen Dyspepsie führt, und Stoffwechselerkrankungen, wie z.B. Stoffwechselsyndrom, beeinträchtigte Glukosetoleranz, Hyperlipidämie, Hypertriglyceridämie, Hypercholesterinämie, niedrige HDL-Konzentrationen und Bluthochdruck.

13. Verbindung nach einem der Ansprüche 1 bis 10 oder ein pharmazeutisch verträgliches Salz davon zur Verwendung bei der Regulation von Sattheit oder der Behandlung von Fettsucht.

14. Verbindung nach einem der Ansprüche 1 bis 10 oder ein pharmazeutisch verträgliches Salz davon zur Verwendung bei der Behandlung von Diabetes.

15. Verbindung nach einem der Ansprüche 1 bis 10 oder ein pharmazeutisch verträgliches Salz davon zur Verwendung bei der Behandlung von Stoffwechselsyndrom (Syndrom X), beeinträchtigter Glukosetoleranz, Hyperlipidämie, Hypertriglyceridämie, Hypercholester-inämie, niedrigen HDL-Konzentrationen oder Bluthochdruck.

16. Verbindung der Formel (XXVII) 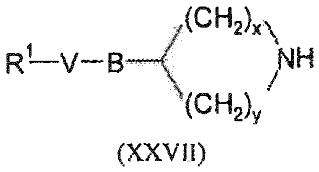 wobei V ein 5-gliedriger Heteroarylring der Formel 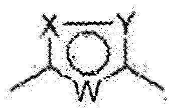 ist,
worin W N ist und einer von X und Y N und der andere O ist,
B (CH₂)ₚ-CH(C₁₋₃-Alkyl)-(CH₂)_{q} ist, worin eine der CH₂-Gruppen durch O, NR⁵, S(O)ₘ, C(O), C(O)NR⁵, CH(NR⁵R⁵⁵), C(O)O, C(O)S, SC(O) oder OC(O) ersetzt sein kann,
m unabhängig 0, 1 oder 2 ist,
p + q gleich 0, 1 oder 2 ist,
x 2 ist,
y 2 ist,
R¹ Phenyl oder eine 6-gliedrige Heteroarylgruppe ist, die bis zu zwei N-Atome enthält, wobei jede davon gegebenenfalls mit einem oder mehreren Substituenten substituiert sein kann, der oder die aus Halogen, C₁₋₄-Alkyl, C₁₋₄-Fluoralkyl, C₁₋₄-Hydroxyalkyl, C₂₋₄-Alkenyl, C₂₋₄-Alkinyl, C₃₋₇-Cycloalkyl, Aryl, OR⁶, CN, NO₂, S(O)ₘR⁶, C(O)NR⁶R⁶⁶, NR⁶R⁶⁶, NR¹⁰C(O)R⁶, NR¹⁰SO₂R⁶, SO₂NR⁶R⁶⁶, COR¹⁰, C(O)OR¹⁰, einer 4- bis 7-gliedrigen Heterocyclylgruppe oder einer 5- oder 6-gliedrigen Heteroarylgruppe ausgewählt ist oder sind, mit der Maßgabe, dass R¹ nicht 4-Fluoralkylpyrid-3-yl oder 4-Fluoralkylpyrimidin-5-yl ist,
R⁵ und R⁵⁵ unabhängig Wasserstoff oder C₁₋₄-Alkyl darstellen,
R⁶ und R⁶⁶ unabhängig Wasserstoff oder C₁₋₄-Alkyl sind, das gegebenenfalls durch Halogen, Hydroxy, C₁₋₄-Alkyloxy-, C₁₋₄-Alkylthio-, C₃₋₇-Heterocyclyl oder N(R¹⁰)₂ substituiert sein kann, oder C₃₋₇-Cycloalkyl, Aryl, Heterocyclyl oder Heteroaryl sind, wobei die cyclischen Gruppen mit einem oder mehreren Substituenten substituiert sein können, der oder die aus Halogen, C₁₋₄-Alkyl, C₁₋₄-Fluoralkyl, OR⁹, CN, SO₂CH₃, N(R¹⁰)₂ und NO₂ ausgewählt ist oder sind, oder R⁶ und R⁶⁶ zusammengenommen einen 5- oder 6-gliedrigen heterocyclischen Ring bilden können, der gegebenenfalls durch Hydroxy, C₁₋₄-Alkyl oder C₁₋₄-Hydroxyalkyl substituiert ist, oder R⁶⁶ C₁₋₄-Alkyloxy- ist, und
R¹⁰ unabhängig Wasserstoff oder C₁₋₄-Alkyl sind oder eine Gruppe N(R¹⁰)₂ einen 4-bis 7-gliedrigen heterocyclischen Ring bilden kann, der gegebenenfalls ein weiteres Heteroatom enthält, das aus O und NR¹⁰ ausgewählt ist.

## Revendications

1. Composé de formule (I) ou sel pharmaceutiquement acceptable de celui-ci : 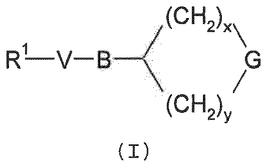 dans laquelle V est un cycle hétéroaryle à 5 chaînons de formule : 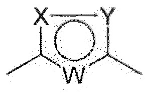 dans laquelle W est N, et l'un de X et Y est N et l'autre est O ;
B est (CH₂)p-CH(alkyle en C₁ à C₃)-(CH₂)_{q}, où l'un des groupes CH₂ peut être remplacé par O, NR⁵, S(O)ₘ C(O), C(O)NR⁵ ou CH(NR⁵R⁵⁵) ;
m vaut indépendamment 0, 1 ou 2 ;
p + q vaut 0, 1 ou 2 ;
x vaut 2;
y vaut 2;
G est NR²;
R¹ est phényle ou un groupe hétéroaryle à 6 chaînons contenant jusqu'à deux atomes N, l'un quelconque de ceux-ci pouvant être éventuellement substitué par un ou plusieurs substituants choisis parmi halogéno, alkyle en C₁ à C₄, fluoroalkyle en C₁ à C₄, hydroxyalkyle en C₁ à C₄, cycloalkyle en C₃ à C₇, OR⁶, CN, S(O)ₘR⁶, C(O)NR⁶R⁶⁶, NR⁶R⁶⁶, N¹⁰C(O)R⁶, NR¹⁰SO₂R⁶, S₂NR⁶R⁶⁶, COR¹⁰, C(O)OR¹⁰, un groupe hétérocyclyle à 4 à 7 chaînons ou un groupe hétéroaryle à 5 ou 6 chaînons ;
R² est C(O) OR³, C(O)NR³R¹³, S(O)₂R³ ou C(O)R³ ; ou un cycle hétéroaryle monocyclique à 5 ou 6 chaînons contenant jusqu'à 4 hétéroatomes choisis parmi N, O et S, qui peuvent éventuellement être substitués par un ou deux groupes choisis parmi alkyle en C₁ à C₄, alcoxy en C₁ à C₄ et halogéno ;
R³ est un alkyle en C₁ à C₈, alcényle en C₂ à C₈ ou alcynyle en C₂ à C₈, l'un quelconque de ceux-ci pouvant éventuellement être substitué par un ou plusieurs atomes d'halogène ou groupes NR⁴R⁴⁴, OR⁴, C(O)OR⁴ , OC(O)R⁴ ou CN, et pouvant contenir un groupe CH₂ qui est remplacé par O ou S ; ou cycloalkyle en C₃ à C₇, aryle, un cycle hétérocyclyle à 4 à 7 chaînons contenant jusqu'à 3 hétéroatomes choisis parmi N, O et S, un cycle hétéroaryle à 6 chaînons contenant jusqu'à 4 hétéroatomes choisis parmi N, O et S, alkylène en C₁ à C₄, cycloalkyle en C₃ à C₇, (alkylène en C₁ à C₄) aryle, (alkylène en C₁ à C₄) hétérocyclyle ou (alkylène en C₁ à C₄) hétéroaryle, l'un quelconque de ceux-ci pouvant être substitué par un ou plusieurs substituants choisis parmi halogéno, alkyle en C₁ à C₄, fluoroalkyle, en C₁ à C₄, OR⁴, CN, NR⁴ R⁴⁴ SO₂Me, NO₂ et C(O)OR⁴;
R⁴ et R⁴⁴ sont indépendamment l'hydrogène ou un alkyle en C₁ à C₄ ; ou bien, pris ensemble, R⁴ et R⁴⁴ peuvent former un cycle hétérocyclique à 5 ou 6 chaînons ;
R⁵ et R⁵⁵ représentent indépendamment l'hydrogène ou un alkyle en C₁ à C₄ ;
R⁶ et R⁶⁶ sont indépendamment l'hydrogène ou un alkyle en C₁ à C₄, qui peut éventuellement être substitué par halogéno, hydroxy, alkyloxy en C₁ à C₄, alkylthio en C₁ à C₄, hétêrocyclyle en C₃ à C₇ ou N(R¹⁰)₂ ; ou cycloalkyle en C₃ à C₇, aryle, hétérocyclyle ou hétéroaryle, où les groupes cycliques peuvent être substitués par un ou plusieurs substituants choisis parmi halogéno, alkyle en C₁ à C₄, fluoroalkyle en C₁ à C₄, OR⁹, CN, SO₂CH₃, N(R¹⁰)₂ et NO₂ ; ou bien, pris ensemble, R⁶ et R⁶⁶ peuvent former un cycle hétérocyclique à 5 ou 6 chaînons éventuellement substitué par hydroxy, alkyle en C₁ à C₄ ou hydroxyalkyle en C₁ à C₄ ; ou encore R⁶⁶ est un alkyloxy en C₁ à C₄ ;
R⁹ est l'hydrogène, un alkyle en C₁ à C₂ ou un fluoroalkyle en C₁ à C₂;
les R¹⁰ sont indépendamment l'hydrogène ou un alkyle en C₁ à C₄ ; ou bien un groupe N(R¹⁰)₂ peut former un cycle hétérocyclique à 4 à 7 chaînons contenant éventuellement un autre hétéroatome choisi parmi O et NR¹⁰ ; et
R¹³ est l'hydrogène ou un alkyle en C₁ à C₄.

2. Composé selon la revendication précédente, ou sel pharmaceutiquement acceptable de celui-ci, dans lequel, dans B, p + q vaut 1 ou 2.

3. Composé selon la revendication 2, ou sel pharmaceutiquement acceptable de celui-ci, dans lequel, dans B, p + q vaut 1.

4. Composé selon l'une quelconque des revendications précédentes, ou sel pharmaceutiquement acceptable de celui-ci, dans lequel B est -CH(CH₃)-O-.

5. Composé selon la revendication 4, ou sel pharmaceutiquement acceptable de celui-ci, dans lequel la configuration absolue au niveau de l'atome de carbone chiral dans -CH(CH₃)-O- est (R).

6. Composé selon l'une quelconque des revendications précédentes, ou sel pharmaceutiquement acceptable de celui-ci, dans lequel R¹ est phényle.

7. Composé selon l'une quelconque des revendications précédentes, ou sel pharmaceutiquement acceptable de celui-ci, dans lequel R² est C(O)OR³, C(O)NR³R¹³ ou un cycle hétéroaryle monocyclique à 5 ou 6 chaînons contenant jusqu'à 4 hétéroatomes choisis parmi N, O et S.

8. Composé selon la revendication 7, ou sel pharmaceutiquement acceptable de celui-ci, dans lequel R² est C(O)OR³.

9. Composé selon l'une quelconque des revendications précédentes, ou sel pharmaceutiquement acceptable de celui-ci, dans lequel R³ représente un alkyle en C₃ à C₅, éventuellement substitué par un ou plusieurs atomes d'halogène ou CN, et peut contenir un groupe CH₂ qui est remplacé par O ou S, ou cycloalkyle en C₃ à C₅, éventuellement substitué par halogéno, alkyle en C₁ à C₄, fluoroalkyle en C₁ à C₄, OR⁴, CN, NR⁴R⁴⁴, NO₂ ou C(O)O (alkyle en C₁ à C₄).

10. Composé de formule (I) choisi parmi les suivants :
ester tert-butylique d'acide 4-{2-[3-(3-fluoro-4-méthylsulfanylphényl)-[1,2,4]oxadiazol-5-yl]-1-méthyl-éthyl}pipéridine-1-carboxylique ;
ester tert-butylique d'acide 4-{2-[3-(3-fluoro-4-méthylsulfanylphényl)-[1,2,4]oxadiazol-5-yl]propyl}-pipéridine-1-carboxylique ;
ester tert-butylique d'acide 4-{1-[3-(3-fluoro-4-méthylsulfanylphényl)-[1,2,4]oxadiazol-5-yl]propoxy}-pipéridine-1-carboxylique ;
ester tert-butylique d'acide 4-{(S)-1-[3-(3-fluoro-4-méthylsulfanylphényl)-[1,2,4]oxadiazol-5-yl]éthoxy}-pipéridine-1-carboxylique ;
ester tert-butylique d'acide 4-{(R)-1-[3-(3-fluoro-4-méthylsulfanylphényl)-[1,2,4]oxadiazol-5-yl]éthoxy}-pipéridine-1-carboxylique;
ester tert-butylique d'acide 4-{1-[3-(3,5-difluoro-4-méthylsulfanylphényl)-[1,2,4]oxadiazol-5-yl]éthoxy}-pipéridine-1-carboxylique ;
1-(3-isopropyl-[1,2,4]oxadiazol-5-yl}-4-{(*R*)-1-[3-(4-méthylsulfanylphényl)-[1,2,4]oxadiazol-5-yl]éthoxy}-pipéridine ;
4-{(*R*)-1-[3-(3-fluoro-4-méthylsulfanylphényl)-[1,2,4]oxadiazol-5-yl]éthoxy}-1-(3-isopropyl-[1,2,4]-oxadiazol-5-yl)pipéridine ;
1-(3-isopropyl-[1,2,4]oxadiazol-5-yl)-4-{(*R*)-1-[3-(3-méthyl-4-méthylsulfanylphényl)-[1,2,4]oxadiazol-5-yl]-éthoxy}pipéridine ;
1-(5-isopropyl-[1,2,4]oxadiazol-3-yl)-4-{(*R*)-1-[3-(4-méthylsulfanylphényl)-[1,2,4]oxadiazol-5-yl]éthoxy}-pipéridine ;
4-{(R)-1-[3-(3-fluoro-4-méthylsulfanylphényl)-[1,2,4]oxadiazol-5-yl]éthoxy}-1-(5-isopropyl-[1,2,4]-oxadiazol-3-yl)pipéridine ;
1-(5-isopropyl-[1,2,4]oxadiazol-3-yl}-4-{(*R*)-1-[3-(3-méthyl-4-méthylsulfanylphényl)-[1,2,4]oxadiazol-5-yl]-éthoxy}pipéridine ;
4-(5-{(*R*)-1-[1-(3-isopropyl-[1,2,4]axadiazol-5-yl)-pipéridin-4-yloxy]éthyl}-[1,2,4]oxadiazol-3-yl)pyridine ;
4-(5-{(*R*)-1-[1-(3-isopropyl-[1,2,4]oxadiazol-5-yl)-pipéridin-4-yloxy]éthyl}-[1,2,4]oxadiazol-3-yl)-2-méthyl-pyridine ;
ester tert-butylique d'acide 4-[(*R*)-1-(3-pyridin-4-yl)-[1,2,4]oxadiazol-5-yl)éthoxy]pipéridine-1-carboxylique ;
ester tert-butylique d'acide 4-[(*R*)-1-[3-(2-méthyl-pyridin-4-yl)-[1,2,4]oxadiazol-5-yl]éthoxyl]pipéridine-1-carboxylique ;
1-(3-tert-butyl-[1,2,4]oxadiazol-5-yl)-4-{(R)-1-[3-(3-fluoro-4-méthylsulfanylphényl)-[1,2,4]oxadiazol-5-yl]-éthoxy}pipéridine ;
ester tert-butylique d'acide 4-{1-[3-(3-fluoro-4-méthanesulfinylphényl)-[1,2,4]oxadiazol-5-yl]propoxy}-pipéridine-1-carboxylique ;
ester tert-butylique d'acide 4-{2-[3-(3-fluoro-4-méthanesulfinylphényl)-[1,2,4]oxadiazol-5-yl]-1-méthyl-éthyl}pipéridine-1-carboxylique ;
ester tert-butylique d'acide 4-{2-[3-(3-fluoro-4-méthanesulfonylphényl)-[1,2,4]oxadiazol-5-yl]-1-méthyl-éthyl}pipéridine-1-carboxylique ;
ester tert-butylique d'acide 4-{2-[3-(3-fluoro-4-méthanesulfinylphényl)-[1,2,4]oxadiazol-5-yl]propyl}-pipéridine-1-carboxylique ;
ester tert-butylique d'acide 4-{2-[3-{3-fluoro-4-méthanesulfonylphényl)-[1,2,4]oxadiazol-5-yl]propyl}-pipéridine-1-carboxylique ;
ester tert-butylique d'acide 4-{(*S*)-1-[3-(3-fluoro-4-méthanesulfonylphényl)-[1,2,4]oxadiazol-5-yl]éthoxy}-pipéridîne-1-carboxylique ;
ester tert-butylique d'acide 4-{(*R*)-1-[3-(3-fluoro-4-méthanesulfonylphényl)-[1,2,4]oxadiazol-5-yl]éthoxy}-pipéridine-1-carboxylique ;
ester tert-butylique d'acide 4-{1-[3-(3-fluoro-4-méthanesulfinylphényl)-[1,2,4]oxadiazol-5-yl]propoxy}-pipéridine-1-carboxylique ;
ester tert-butylique d'acide 4-{1-[3-(3-fluoro-4-méthanesulfonylphényl)-[1,2,4]oxadiazol-5-yl]propoxy}-pipéridine-1-carboxylique ;
ester tert-butylique d'acide 4-{1-[3-(3,5-difluoro-4-méthanesulfinylphényl)-[1,2,4]oxadiazol-5-yl]éthoxy}-pipéridine-1-carboxylique ;
ester tert-butylique d'acide 4-{1-[3-(3,5-difluoro-4-méthanesulfonylphényl)-[1,2,4]oxadiazol-5-yl]éthoxy}-pipéridine-1-carboxylique ;
1-(3-tert-butyl-[1,2,4]oxadiazol-5-yl)-4-{(*R*)-1-[3-(3-fluoro-4-méthanesulfinylphényl)-[1,2,4]oxadiazol-5-yl]-éthoxy}pipéridine ;
1-{3-tert-butyl-[1,2,4]oxadiazol-5-yl)-4-{(*R*)-1-[3-(3-fluoro-4-méthanesulfonylphényl)-[1,2,4]oxadiazol-5-yl]-éthoxy}pipéridine ;
1-(3-isopropyl-[1,2,4]oxadiazol-5-yl)-4-{(*R*)-1-[3-(4-méthanesulfonylphényl)-[1,2,4]oxadiazol-5-yl]éthoxy}-pipéridine ;
4-{(*R*)-1-[3-(3-fluoro-4-méthanesulfonylphényl)-[1,2,4]oxadiazol-5-yl]éthoxy}-1-(3-isopropyl-[1,2,4]-oxadiazol-5-yl)pipéridine ;
1-(3-isopropyl-[1,2,4]oxadiazol-5-yl)-4-{(*R*)-1-[3-(4-méthanesulfonyl-3-méthylphényl)-[1,2,4]oxadiazol-5-yl]-éthoxy}pipéridine ;
1-(5-isopropyl-[1,2,4]oxadiazol-5-yl)-4-{(*R*)-1-[3-(4-méthanesulfonylphényl)-[1,2,4]oxadiazol-5-yl]éthoxy}-pipéridine ;
4-{(*R*)-1-[3-(3-fluoro-4-méthanesulfonylphényl)-[1,2,4]oxadiazol-5-yl]éthoxy}-1-(5-isopropyl-[1,2,4]-oxadiazol-3-yl)pipéridine ;
1-(5-isopropyl-[1,2,4]oxadiazol-3-yl)-4-{(*R*)-1-[3-(4-méthanesulfonyl-3-méthylphényl)-[1,2,4]oxadiazol-5-yl]-éthoxy}pipéridine ;
ester tert-butylique d'acide 4-{1-[3-(3-fluoro-4-sulfamoylphenyl)-[1,2,4]oxadiazol-5-yl]-éthoxy}pipéridine-1-carboxylique ;
ester tert-butylique d'acide 4-{(*R*}-1-[3-(3-fluoro-4-sulfamoylphényl)-[1,2,4]oxadiazol-5-yl]éthoxy}pipéridine-1-carboxylique ;
ester isopropylique d'acide 4-{(*R*)-1-[3-(3-fluoro-4-méthanesulfonylphényl)-[1,2,4]oxadiazol-5-yl]éthoxy}-pipéridine-1-carboxylique ;
ester tert-butylique d'acide 4-{1-[3-(4-méthane-sulfonylphényl)-[1,2,4]oxadiazol-5-yl]éthoxy}pipéridine-1-carboxylique ;
ester tert-butylique d'acide 4-{1-[3-(3-fluoro-4-méthylsulfanylphényl)-[1,2,4]oxadiazol-5-yl]éthoxy}-pipéridine-1-carboxylique ;
ester tert-butylique d'acide 4-{1-[3-(3-fluoro-4-méthoxycarbonylphényl)-[1,2,4]oxadiazol-5-yl]éthoxy}-pipéridine-1-carboxylique ;
ester tert-butylique d'acide 4-{1-[3-(4-carbamoyl-3-fluorophényl)-[1,2,4]oxadiazol-5-yl]éthoxy}pipéridine-1-carboxylique ;
ester tert-butylique d'acide 4-{1-[3-(4-éthyl-carbamoyl-3-fluorophényl)-[1,2,4]oxadiazol-5-yl]éthoxy}-pipéridine-1-carboxylique ;
ester tert-butylique d'acide 4-{1-[3-(3-fluoro-4-méthanesulfonylphényl)-[1,2,4]oxadiazol-5-yl]éthoxy}-pipéridine-1-carboxylique ; et
ester tert-butylique d'acide 4-{1-[3-(3-fluoro-4-méthanesulfinylphényl)-[1,2,4]oxadiazol-5-yl]éthoxy}-pipéridine-1-carboxylique ;
et les sels pharmaceutiquement acceptables de n'importe lesquels de ceux-ci.

11. Composition pharmaceutique comprenant un composé selon l'une quelconque des revendications 1 à 10, ou un sel pharmaceutiquement acceptable de celui-ci, et un véhicule pharmaceutiquement acceptable.

12. Composé selon l'une quelconque des revendications 1 à 10, ou sel pharmaceutiquement acceptable de celui-ci, pour utilisation dans le traitement du diabète ; de l'obésité ; du syndrome métabolique ; du diabète de type 1 et de type 2 ; d'une altération de la tolérance au glucose ; d'une résistance à l'insuline ; de complications diabétiques telles qu'une neuropathie, une néphropathie, une rétinopathie, une cataracte, des complications cardiovasculaires, une dyslipidémie ; d'une sensibilité anormale aux graisses ingérées conduisant à une dyspepsie fonctionnelle ; et de maladies métaboliques telles que le syndrome métabolique, une altération de la tolérance au glucose, l'hyperlipidémie, l'hypertriglycéridémie, l'hypercholestérolémie, les niveaux bas de HDL, et l'hypertension.

13. Composé selon l'une quelconque des revendications 1 à 10, ou sel pharmaceutiquement acceptable de celui-ci, pour utilisation dans la régulation de la satiété ou le traitement de l'obésité.

14. Composé selon l'une quelconque des revendications 1 à 10, ou sel pharmaceutiquement acceptable de celui-ci, pour utilisation dans le traitement du diabète.

15. Composé selon l'une quelconque des revendications 1 à 10, ou sel pharmaceutiquement acceptable de celui-ci, pour utilisation dans le traitement du syndrome métabolique (syndrome X), d'une altération de la tolérance au glucose, de l'hyperlipidémie, de l'hypertriglycéridémie, de l'hypercholestérolémie, de niveaux bas de HDL, ou de l'hypertension.

16. Composé de formule (XXVII) : 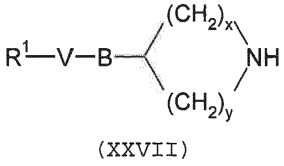 dans laquelle V est un cycle hétéroaryle à 5 chaînons de formule : 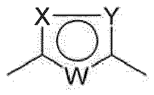 dans laquelle W est N, et l'un de X et Y est N et l'autre est O ;
B est (CH₂)ₚ-CH(alkyle en C₁ à C₃) - (CH₂)_{q}, où l'un des groupes CH₂ peut être remplacé par O, NR⁵, S(O)ₘ, C(O), C(O)NR⁵, CH(NR⁵R⁵⁵), C(O)O, C(O)S, SC(O) ou OC(O) ;
m vaut indépendamment 0, 1 ou 2 ;
p + q vaut 0, 1 ou 2 ;
x vaut 2 ;
y vaut 2 ;
R¹ est phényle ou un groupe hétéroaryle à 6 chaînons contenant jusqu'à deux atomes N, l'un quelconque de ceux-ci pouvant être éventuellement substitué par un ou plusieurs substituants choisis parmi halogéno, alkyle en C₁ à C₄, fluoroalkyle en C₁ à C₄, hydroxyalkyle en C₁ à C₄, alcényle en C₂ à C₄, alcynyle en C₂ à C₄, cycloalkyle en C₃ à C₇, aryle, OR⁶, CN, NO₂, S (O) ₘR⁶, C(O)NR⁶R⁶⁶, NR⁶R⁶⁶, NR¹⁰C(O)R⁶, NR¹⁰SO₂R⁶, SO₂NR⁶R⁶⁶, COR¹⁰, C(O)OR¹⁰, un groupe hétérocyclyle à 4 à 7 chaînons ou un groupe hétéroaryle à 5 ou 6 chaînons, à condition que R¹ ne soit pas un 4-fluoroalkylpyrid-3-yle ou un 4-fluoroalkylpyrimidin-5-yle ;
R⁵ et R⁵⁵ représentent indépendamment l'hydrogène ou un alkyle en C₁ à C₄ ;
R⁶ et R⁶⁶ sont indépendamment l'hydrogène ou un alkyle en C₁ à C₄, qui peut éventuellement être substitué par halogéno, hydroxy, alkyloxy en C₁ à C₄, alkylthio en C₁ à C₄, hétérocyclyle en C₃ à C₇ ou N(R¹⁰)₂ ; ou cycloalkyle en C₃ à C₇, aryle, hétérocyclyle ou hétéroaryle, où les groupes cycliques peuvent être substitués par un ou plusieurs substituants choisis parmi halogéno, alkyle en C₁ à C₄, fluoroalkyle en C₁ à C₄, OR⁹, CN, SO₂CH₃, N(R¹⁰)₂ et NO₂ ; ou bien, pris ensemble, R⁶ et R⁶⁶ peuvent former un cycle hétérocyclique à 5 ou 6 chaînons éventuellement substitué par hydroxy, alkyle en C₁ à C₄ ou hydroxyalkyle en C₁ à C₄ ; ou encore R⁶⁶ est un alkyloxy en C₁ à C₄ ; et les R¹⁰ sont indépendamment l'hydrogène ou un alkyle en C₁ à C₄ ; ou bien un groupe N(R¹⁰)₂ peut former un cycle hétérocyclique à 4 à 7 chaînons contenant éventuellement un autre hétéroatome choisi parmi O et NR¹⁰.
